# EUROPEAN PATENT APPLICATION

(11) **EP 4 671 368 A1**
(43) Date of publication of application: **31.12.2025**
(21) Application number: 24759795.8
(22) Date of filing: 24.02.2024
(51) Int. Cl.: C12N 9/12, C12N 9/22, C12N 15/90, C12N 15/54, C12N 15/33, C12P 19/34

(54) **ENZYME CAPABLE OF MEDIATING INTEGRATION OF LARGE DNA FRAGMENT INTO MAMMALIAN GENOME AND USE THEREOF**

(30) Priority: 24.02.2023 CN 202310168103
(71) Applicant: Yoltech Therapeutics Co., Ltd, Shanghai 201109 (CN)
(72) Inventor: LI, Zexu, Shanghai 201109 (CN); ZHANG, Hexin, Shanghai 201109 (CN)
(74) Representative: CH Kilger Anwaltspartnerschaft mbB
(86) International application number: PCT/CN2024/078465
(87) International publication number: WO 2024/175123

(57) **Abstract**

The disclosure relates to an enzyme capable of mediating the integration of large DNA fragments into mammalian genomes and uses thereof. Specifically, novel enzymes that can integrate large DNA fragments into genomes are provided, including integrases as shown in SEQ ID NO: 1-7. The disclosure further relates to polynucleotides containing nucleotide sequences encoding the integrases, and host cells containing these polynucleotides. The disclosure also relates to the integrases and methods for recombining target nucleic acids into the human genome by using the integrases.

## Description

### Technical Field

The disclosure relates to the field of gene editing, and specifically, to enzymes capable of mediating the integration of large-fragment DNA into mammalian genomes and uses thereof.

### Background Art

Integrase is a recombinase derived from bacteriophages, and can mediate recombination between the attP site on the bacteriophage genome and the attB site on the bacterial genome, thereby site-specifically integrating the bacteriophage genome into the host genome. Studies have shown that mammalian genomes have pseudo attP sites that are homologous to the attP site, and these pseudo attP sites can also recombine with a plasmid carrying the attB site under the action of integrase, thereby integrating an exogenous gene on a plasmid into a genome.

PhiC31 integrase is an integrase derived from the PhiC31 bacteriophage, and can mediate recombination between the attP site on the circular genome of the bacteriophage itself and the attB site on the circular genome of *Streptomyces lividans.* Using this principle, researchers first integrated a plasmid containing the attB site sequence into genomes of bacteria, yeast, and mammalian cells containing the attP site, under the catalysis of PhiC31 integrase. This method has been used in preclinical studies on hemophilia A and hemophilia B, and the results showed that it can effectively increase the coagulation factor level in hemophilia A and hemophilia B mice, achieving long-term improvement of disease symptoms. Integrase has the characteristics of unidirectional integration, relatively specific target sites, no need for cofactors, and capability of mediating large-fragment integration, thus showing a great application potential in the field of gene therapy.

However, existing integrases have some drawbacks, such as low integration efficiency in the human genome, multi-site integration, and potential risks such as chromosomal rearrangement caused thereby.

Therefore, there is an urgent need in the art to develop new integrases with advantages such as high integration efficiency.

### Summary of the Disclosure

The disclosure provides enzymes capable of mediating integration of large-fragment DNA into mammalian genomes and uses thereof.

In a first aspect of the present disclosure, an integrase is provided, wherein the integrase is selected from the group consisting of:
(a) an integrase represented by any one of SEQ ID NOs: 1-7;
(b) a polypeptide with an amino acid sequence having at least 80%, at least 85%, at least 90%, at least 91%, at least 92%, at least 93%, at least 94%, at least 95%, at least 96%, at least 97%, at least 98%, or at least 99% sequence identity to the amino acid sequence represented by any one of SEQ ID NOs: 1-7, and substantially retaining the biological function of any one of SEQ ID NOs: 1-7;
(c) a polypeptide derived from (a), formed by substitution, deletion, or addition of one or more amino acid residues in the amino acid sequence represented by any one of SEQ ID NOs: 1-7, and having an integrase function.

In another preferred embodiment, the amino acid sequence of the integrase is represented by any one of SEQ ID NOs: 1-7.

In another preferred embodiment, the biological function is the function of integrating an exogenous gene into a genome.

In another preferred embodiment, the integrase is Cyin14.

In a second aspect of the disclosure, a polynucleotide is provided, wherein the polynucleotide encodes the integrase according to the first aspect of the disclosure.

In another preferred embodiment, the nucleotide sequence of the polynucleotide is represented by SEQ ID NOs: 10-16.

In a third aspect of the disclosure, a vector is provided, wherein the vector comprises the polynucleotide according to the second aspect of the disclosure.

In another preferred embodiment, the polynucleotide molecule is operably linked to a regulatory sequence to allow expression of the polynucleotide molecule.

In another preferred embodiment, the regulatory sequence includes a promoter sequence.

In a fourth aspect of the disclosure, a genetically engineered host cell is provided, which contains the vector according to the third aspect of the disclosure, or has the polynucleotide according to the second aspect of the disclosure integrated in its genome.

In another preferred embodiment, the host cell expresses the integrase according to the first aspect of the disclosure.

In another preferred embodiment, the host cell includes eukaryotic cells and prokaryotic cells.

In another preferred embodiment, the host cell is a mammalian cell.

In a fifth aspect of the disclosure, a method for preparing the integrase according to the first aspect of the disclosure is provided, the method comprising:
(a) culturing the host cell according to the fourth aspect of the disclosure under a condition suitable for expression; and
(b) isolating the integrase from the culture.

In a sixth aspect of the disclosure, a cell engineered with the integrase of the disclosure is provided, wherein the cell comprises:
(i) an integration sequence selected from the group consisting of: a nucleotide sequence represented by any one of SEQ ID NOs: 17-42, a nucleotide sequence having at least 80%, 85%, 90%, 95%, 96%, 97%, 98%, or 99% identity thereto, or a nucleotide sequence having not more than 1, 2, 3, 4, 5, 6, 7, or 8 sequence alterations (e.g., substitutions, insertions, or deletions) relative to the nucleotide sequence represented by any one of SEQ ID NOs: 17-42; and
(ii) a heterologous target nucleic acid.

In another preferred embodiment, the cell is a eukaryotic cell or a prokaryotic cell.

In another preferred embodiment, the cell is a eukaryotic cell.

In another preferred embodiment, the cell is a human or non-human mammalian cell.

In another preferred embodiment, the heterologous target nucleic acid is integrated in the genome of the cell via the integrase of the first aspect of the disclosure.

In another preferred embodiment, the integrase is Cyin14.

In another preferred embodiment, when the integrase is Cyin14, the corresponding integration sequence contains integration sequence 14-P1 (SEQ ID NO: 31).

In a seventh aspect of the disclosure, a recombination method is provided, the method comprising:
(a) providing a target nucleic acid to be integrated and a host genome;
(b) in the presence of the integrase according to the first aspect of the disclosure, allowing the target nucleic acid to be integrated into the genome.

In another preferred embodiment, the target nucleic acid to be integrated contains one or more recognition sequences (or integration sequences).

In another preferred embodiment, the recognition sequence is located upstream (or immediately upstream), downstream (or immediately downstream), or on both flanks of the target nucleic acid sequence to be integrated.

In another preferred embodiment, the recognition sequence (or integration sequence) on the target nucleic acid to be integrated and a recognition sequence (or a pseudo-recognition sequence) in the genome are capable of mediating site-specific integration under the action of the integrase according to the first aspect of the disclosure.

In another preferred embodiment, the pseudo-recognition sequence is located in or near a genomic safe harbor (GSH) site.

In another preferred embodiment, in step (b), the target nucleic acid, the genome, and the integrase are brought into contact.

In another preferred embodiment, in step (b), the target nucleic acid, a cell containing the genome, and the integrase are brought into contact.

In another preferred embodiment, the method includes *in vitro* methods and *in vivo* methods.

In another preferred embodiment, the method is non-diagnostic and non-therapeutic.

In an eighth aspect of the disclosure, a reaction system for gene integration is provided, the reaction system comprising:
(a) a target nucleic acid to be integrated;
(b) a host genome; and
(c) the integrase according to the first aspect of the disclosure.

In a ninth aspect of the disclosure, a kit for detecting a target nucleic acid in a sample is provided, the kit comprising:
(a) the integrase according to the first aspect of the disclosure;
(b) the polynucleotide according to the second aspect of the disclosure; or
(c) the vector according to the third aspect of the disclosure.

In a tenth aspect of the disclosure, provided is a use of the integrase according to the first aspect of the disclosure, the polynucleotide according to the second aspect of the disclosure, the vector according to the third aspect of the disclosure, or the host cell according to the fourth aspect of the disclosure in the manufacture of a reagent or kit, wherein the reagent or kit is for use in:
(i) gene or genome editing; or
(ii) editing a target sequence in a target locus to modify an organism or a non-human organism.

In an eleventh aspect of the disclosure, a method for site-specific integration of an exogenous nucleic acid into the genome or an intracellular target nucleic acid of a cell is provided, the method comprising:
(a) introducing into a cell (i) a first construct, (ii) a gRNA, (iii) an exogenous nucleic acid to be integrated, and (iv) a second construct,
   (i) the first construct, which is an expressible polynucleotide construct encoding an editing polypeptide, wherein the editing polypeptide comprises a DNA-binding nuclease domain linked via a linker to a reverse transcriptase domain, and wherein the DNA-binding nuclease domain comprises nickase activity; and
   (ii) the gRNA, which is a guide RNA comprising a targeting sequence, a primer binding sequence, and a complementary sequence to an integration sequence, wherein the gRNA interacts with the expressed editing polypeptide to target the editing polypeptide to a specific target site in the genome or the intracellular target nucleic acid of the cell, and the DNA-binding nuclease domain of the editing polypeptide cleaves one strand of the genome or the intracellular target nucleic acid of the cell, and the reverse transcriptase domain incorporates the integration sequence into the cleaved site, thereby incorporating at least one integration sequence into a specific target site in the genome or the target nucleic acid of the cell; and
   (iii) the exogenous nucleic acid to be integrated, which is linked to a recognition sequence homologous to the integration sequence, wherein in the presence of both the recognition sequence and the integration sequence, the integrase mediates site-specific integration; and
   (iv) the second construct, which is an expressible polynucleotide construct encoding the integrase according to the first aspect of the disclosure, wherein the integrase integrates the exogenous nucleic acid into the specific target site of the genome of the cell;
(b) in the presence of the editing polypeptide, the gRNA, the integrase, and the exogenous nucleic acid to be integrated, allowing the integrase to integrate the exogenous nucleic acid into the genome or the intracellular target nucleic acid of the cell.

In another preferred embodiment, (i) the first construct, (ii) the gRNA, (iii) the exogenous nucleic acid to be integrated, and (iv) the second construct may be introduced into the cell independently of each other, in batches, or simultaneously.

In another preferred embodiment, the integration sequence is a sequence selected from the group consisting of SEQ ID NOs: 17-42, and 47-48.

In another preferred embodiment, (i) the first construct, (ii) the gRNA, and (iv) the second construct are integrated into the cell genome by virus, lipid, microvesicle, gene gun, or a combination thereof.

In another preferred embodiment, the gRNA hybridizes to a complementary strand in the cell genome.

In another preferred embodiment, the exogenous nucleic acid to be integrated is integrated into the cell genome via minicircle DNA, plasmid, mRNA, linear DNA, or a combination thereof.

In another preferred embodiment, the DNA-binding nuclease domain having nickase activity is selected from the group consisting of nickase of Cas9, nickase of Cas12a, nickase of Cas12b, nickase of Cas12c, nickase of Cas12d, nickase of Cas12e, nickase of Cas12f, nickase of Cas12g, nickase of Cas12h, nickase of Cas12i, nickase of Cas12j, nickase of Cas12k, nickase of Cas12l, nickase of Cas12m, nickase of Cas12n, or a combination thereof.

In another preferred embodiment, the reverse transcriptase domain comprises a mutation relative to the wild-type sequence.

In another preferred embodiment, the reverse transcriptase domain is selected from the group consisting of the Moloney murine leukemia virus (M-MLV) reverse transcriptase domain, transcriptional heteropolymerase (RTX), avian myeloblastosis virus reverse transcriptase (AMV-RT), *Eubacterium rectale* maturase RT (MarathonRT), or a combination thereof.

In another preferred embodiment, the M-MLV reverse transcriptase domain comprises a mutation selected from the group consisting of D200N, T306K, W313F, T330P, L603W, or a combination thereof.

In another preferred embodiment, the exogenous nucleic acid to be integrated is selected from the group consisting of β-globin (HBB) gene, genes causing β-thalassemia or sickle cell anemia, metabolic genes, genes related to infectious diseases, genes related to hereditary diseases, or a combination thereof.

In a twelfth aspect of the disclosure, a polypeptide, such as a fusion protein, is provided, comprising a DNA-binding nuclease and the integrase according to the first aspect of the disclosure or an active fragment thereof.

In another preferred embodiment, the fusion protein comprises:
a) the integrase according to the first aspect of the disclosure or an active fragment thereof, having a first amino acid sequence;
b) a CRISPR-associated (Cas) protein or an active fragment thereof, having a second amino acid sequence; and
c) a nuclear localization signal (NLS) having a third amino acid sequence.

In another preferred embodiment, the fusion protein comprises a DNA-binding nuclease, the integrase according to the first aspect, and a reverse transcriptase.

In another preferred embodiment, the fusion protein comprises, from N-terminus to C-terminus, a DNA-binding nuclease, a reverse transcriptase, and the integrase according to the first aspect.

In another preferred embodiment, the reverse transcriptase is linked to the integrase according to the first aspect of the disclosure via a linker.

In another preferred embodiment, the DNA-binding nuclease is linked to the reverse transcriptase via a linker.

In another preferred embodiment, the linker is cleavable or non-cleavable.

In another preferred embodiment, the DNA-binding nuclease has nickase activity.

In another preferred embodiment, the DNA-binding nuclease is selected from the group consisting of Cas9, Cas12a, Cas12b, Cas12c, Cas12d, Cas12e, Cas12f, Cas12g, Cas12h, Cas12i, Cas12j, Cas12k, Cas12l, Cas12m, Cas12n, or a combination thereof.

In another preferred embodiment, the reverse transcriptase is selected from the group consisting of the Moloney murine leukemia virus (M-MLV) reverse transcriptase domain, transcriptional heteropolymerase (RTX), avian myeloblastosis virus reverse transcriptase (AMV-RT), *Eubacterium rectale* maturase RT (MarathonRT), or a combination thereof.

In another preferred embodiment, the reverse transcriptase is an M-MLV reverse transcriptase modified relative to the wild-type M-MLV reverse transcriptase.

In another preferred embodiment, the M-MLV reverse transcriptase domain comprises a mutation selected from the group consisting of D200N, T306K, W313F, T330P, L603W, or a combination thereof.

In a thirteenth aspect of the disclosure, a sequence integration system is provided, the integration system comprising:
(i) a first construct, which is an expressible polynucleotide construct encoding an editing polypeptide, wherein the editing polypeptide comprises a DNA-binding nuclease domain linked via a linker to a reverse transcriptase domain, and wherein the DNA-binding nuclease domain comprises nickase activity;
(ii) a gRNA, which is a guide RNA comprising a targeting sequence, a primer binding sequence, and a complementary sequence to an integration sequence,
   wherein the gRNA interacts with the expressed editing polypeptide to target the editing polypeptide to a specific target site in the genome or an intracellular target nucleic acid of a cell, and the DNA-binding nuclease domain of the editing polypeptide cleaves one strand of the genome or the intracellular target nucleic acid of the cell, and the reverse transcriptase domain incorporates the integration sequence into the cleaved site, thereby incorporating at least one integration sequence into the specific target site in the genome or the target nucleic acid of the cell;
(iii) an exogenous nucleic acid to be integrated, which is linked to a recognition sequence homologous to the integration sequence, and the recognition sequence and the integration sequence are capable of mediating site-specific integration; and
(iv) a second construct, which encodes the integrase according to the first aspect of the disclosure,
   wherein the integrase integrates the exogenous nucleic acid into the specific target site in the cell genome.

In another preferred embodiment, the integration is an *in vitro* method.

In a fourteenth aspect of the disclosure, a vector is provided, comprising a nucleic acid encoding the fusion protein according to the twelfth aspect of the disclosure.

In a fifteenth aspect of the disclosure, a gRNA specifically binding to a DNA-binding nuclease is provided, comprising (a) a primer binding site that hybridizes to a nicked DNA strand; (b) an integration sequence for an integrase; and (c) a targeting sequence.

In another preferred embodiment, the integrase is Cyin14.

In another preferred embodiment, when the integrase is Cyin14, the corresponding integration sequence contains integration sequence 14-P1 (SEQ ID No: 31).

In a sixteenth aspect of the disclosure, a therapeutic method is provided, which comprises integrating an exogenous nucleic acid to be integrated into the genome of a subject in need by the method according to the twelfth aspect of the disclosure.

In another preferred embodiment, the subject includes a human or a non-human mammal.

It should be understood that within the scope of the disclosure, the above technical features of the disclosure and the technical features specifically described in the following description (such as examples) can be combined with each other to form new or preferred technical solutions, and the detailed descriptions thereof are omitted here.

### Brief Description of Drawings

Figure 1 shows the functional domains of integrases Cyin10, Cyin11, Cyin12, Cyin13, Cyin14, Cyin15, and Cyin16.
Figure 2 shows the three-dimensional structures of integrases Cyin10, Cyin11, Cyin12, Cyin13, Cyin14, Cyin15, and Cyin16.
Figure 3 shows a flow chart of the integration experiment.
Figure 4 shows that the ABE8e nucleotide sequence at positions 409-5226 in an ABE8e plasmid has been replaced with, for example, Cyin10; other expression plasmids are constructed in the same manner.

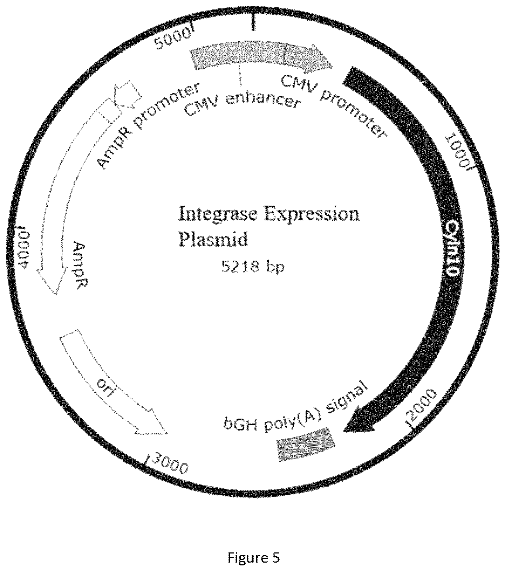

Figure 5 shows a donor plasmid expressing EGFP, where the EGFP nucleotide sequence is located between the Hindlll and Kpnl restriction sites.
Figure 6 shows the integration efficiency of 7 different mediatable donor plasmids into the genome of 293T cells.
Figure 7 shows the flow cytometry analysis results of integrases Cyin14 and Cyin16, the flow cytometry assay result of integrase phiC31, and the flow cytometry assay result of a control group.
Figure 8 shows a comparison of integration efficiency between integrases Cyin14, Cyin16 and the reported phiC31 and Bxb1.
Figure 9 shows the phylogenetic tree of integrases Cyin10, Cyin11, Cyin12, Cyin13, Cyin14, Cyin15, and Cyin16.

### Detailed Description of Invention

After extensive and in-depth research and large-scale screening, the inventors have developed integrases with novel structures for the first time. The integrases of the disclosure have low homology with existing integrases but exhibit excellent integration activity, particularly in mediating integration of large-fragment DNA into mammalian genomes. The disclosure is completed based on this.

### Definition

The terms used in the technical solutions of the disclosure are explained as follows:
Operably linked: It refers to the connection of a target nucleotide sequence to regulatory elements in a manner that allows expression of the nucleotide sequence (e.g., in an *in vitro* transcription/translation system or in a host when the vector is introduced into a host cell).
attB/attP reaction: Also known as B/P reaction, it refers to a mediated recombination reaction between the attB recognition site and the attP recognition site.
att site: A site on a DNA molecule for attachment of an integrase or integrase complex.
Integrated: It refers to the integration of an exogenous gene or nucleotide sequence into a host genome through covalent bonds to the host DNA.
Recognition site: Also known as a recombination site, it refers to a nucleotide sequence that can be recognized by a recombinase protein.
Target sequence: Also known as target DNA, it refers to a nucleotide sequence containing at least one recombinase recognition site. A target nucleotide sequence may be a gene, an expression cassette, a promoter, a molecular marker, a part of any of these, etc.
Site-specific recombination: Also known as sequence-specific recombination, it refers to recombination between two nucleotide sequences each containing at least one recognition site or at least one non-homologous site.

"Site-specific" means happening at a specific nucleotide sequence, e.g., at a specific position in the genome of a host cell. The nucleotide sequence may be endogenous to the host cell, located at a natural position in the host genome or at another position in the genome, or it may be a heterologous nucleotide sequence inserted into the genome of the host cell by any of a variety of known methods.

Host cell: It refers to a cell into which a protein and/or genetic material has been introduced. It refers not only to a specific subject cell, but also to the progeny of the cell. Host cells may be isolated cells, a cell line, or living tissue grown in a culture. Host cells include animal cells or plant cells, e.g., human cells, non-human mammalian cells, insect cells, avian cells, rodent cells, maize cells, soybean cells, wheat cells, or rice cells.

Target nucleic acid: "Target nucleic acid" is used interchangeably with "target gene", "target sequence", or "target DNA", and includes any nucleotide sequence that, when transferred into a cell, confers a desired trait on the cell. The desired trait includes, but is not limited to: viral resistance, insect resistance, antibiotic stress resistance, disease resistance, resistance to other pests, herbicide tolerance, improved nutritional value, improved performance in industrial processes, or altered reproductive capacity, etc. A target nucleic acid may also be a sequence transferred into a cell line or a mammal or a plant for production of a commercially valuable enzyme or metabolite.

As used herein, "target nucleic acid to be integrated", "exogenous nucleic acid to be integrated", "nucleic acid to be integrated", "nucleic acid of interest to be integrated", "target gene to be integrated", "target sequence to be integrated", and "target DNA to be integrated" are used interchangeably and all refer to a target nucleic acid to be integrated into a cell genome, preferably, the nucleic acid is heterologous.

As used herein, "heterologous" refers to DNA or RNA that has a different origin from the cell or cell genome into which it is to be integrated.

As used herein, the terms "recognition sequence" or "integration sequence" are used interchangeably and generally refer to a nucleic acid (e.g., DNA) sequence recognized by a recombinase polypeptide (e.g., capable of being bound by the recombinase polypeptide). For example, as described herein, in some cases, there are two recognition sequences, of which one located at the integration site (the site where a nucleic acid is to be integrated) and the other adjacent to the nucleic acid of interest to be introduced at the integration site. Recognition sequences are generally referred to as attB and attP. Recognition sequences may be natural, or modified relative to natural sequences. The length of a recognition sequence can vary but is typically about 20 to about 200 nt, about 30 to 90 nt, more usually 30 to 70 nucleotides. Typically, the target nucleic acid to be integrated contains one or more recognition sequences (or integration sequences). The recognition sequence(s) can be located upstream (or immediately upstream), downstream (or immediately downstream), or on both flanks of the target nucleic acid sequence to be integrated. The recognition sequence in a recombinant vector cooperates with the recognition sequence (or integration sequence) in the genome to integrate the target nucleic acid sequence to be integrated at a predetermined position. It should be understood that recognition sequences exist in the genomes of various organisms, where a recognition sequence does not necessarily have the same nucleotide sequence as the wild-type recognition sequence (for a given recombinase); however, such natural recognition sequences are still sufficient to promote recombinase-mediated recombination.

### Integrases and Their Applications

In the present disclosure, isolated novel integrases are provided, including Cyin10, Cyin11, Cyin12, Cyin13, Cyin14, Cyin15, Cyin16, and their derived polypeptides with the same or substantially the same integrase activity. The disclosure also provides uses of these novel integrases, particularly use in mediating the integration of large-fragment DNA into a mammalian genome.

As used herein, "isolated" means that a substance is separated from its original environment (if it is a natural substance, the original environment is its natural environment). For example, polynucleotides and polypeptides in their natural state within living cells are not isolated or purified, but the same polynucleotides or polypeptides are considered isolated or purified if they are separated from the other substances also present in the natural state.

As used herein, "an isolated integrase or polypeptide" means that the integrase polypeptide is substantially free of other proteins, lipids, carbohydrates, or other substances naturally associated with it. Those skilled in the art can purify integrases using standard protein purification techniques. A substantially pure polypeptide produces a single major band on a non-reducing polyacrylamide gel. The purity of an integrase polypeptide can be analyzed by amino acid sequence analysis.

The polypeptides of the disclosure may be recombinant polypeptides, natural polypeptides, or synthetic polypeptides, preferably recombinant polypeptides. The polypeptides of the disclosure may be purified natural products, chemically synthesized products, or produced using recombinant technology from prokaryotic or eukaryotic hosts (e.g., bacteria, yeast, higher plants, insects, and mammalian cells). Depending on the host used in the recombinant production protocol, the polypeptides of the disclosure may be glycosylated or non-glycosylated. The polypeptides of the disclosure may also include or not include an initial methionine residue.

The disclosure also includes fragments, derivatives, and analogs of the integrases. As used herein, the terms "fragment", "derivative", and "analog" refer to polypeptides that substantially retain the same biological function or activity as that of the natural integrases of the disclosure. The polypeptide fragment, derivative, or analog of the disclosure may be (i) a polypeptide with one or more conservative or non-conservative amino acid residues (preferably conservative amino acid residues) substituted, wherein such substituted amino acid residues may or may not be encoded by a genetic code; or (ii) a polypeptide with substituent groups in one or more amino acid residues; or (iii) a polypeptide formed by fusing a mature polypeptide with another compound (such as a compound that extends the half-life of the polypeptide, e.g., polyethylene glycol); or (iv) a polypeptide formed by fusing an additional amino acid sequence to this polypeptide sequence (such as a leader sequence, a secretion sequence, a sequence used to purify the polypeptide, a proprotein sequence, or a fusion protein formed with an antigenic IgG fragment). These fragments, derivatives, and analogs are within the scope well known to those skilled in the art based on the teachings herein.

In the disclosure, the term "integrase polypeptide" refers to a polypeptide having the amino acid sequence represented by any one of SEQ ID NOs: 1-7 and exhibiting integrase activity. This term also includes variant forms of the amino acid sequence represented by any one of SEQ ID NOs: 1-7 that have the same function as the integrase. These variant forms include, but are not limited to, deletion, insertion, and/or substitution of one or more (usually 1-50, preferably 1-30, more preferably 1-20, most preferably 1-10) amino acids, and addition of one or several (usually within 20, preferably within 10, more preferably within 5) amino acids at the C-terminus and/or N-terminus. For example, in the art, substitution with amino acids having similar or analogous properties usually does not alter the function of the protein. For another example, addition of one or several amino acids at the C-terminus and/or N-terminus usually does not alter the function of the protein either. This term also includes active fragments and active derivatives of the integrase.

The variant forms of the polypeptide include: homologous sequences, conservative variants, allelic variants, natural mutants, induced mutants, proteins encoded by DNAs that hybridize with integrase DNA under high or low stringency conditions, and polypeptides or proteins obtained using antisera against the integrase polypeptide. The disclosure also provides other polypeptides, such as fusion proteins containing the integrase polypeptide or a fragment thereof. In addition to nearly full-length polypeptides, the disclosure also includes soluble fragments of the integrase polypeptide. Typically, such fragments have at least about 10 consecutive amino acids, usually at least about 30 consecutive amino acids, preferably at least about 50 consecutive amino acids, more preferably at least about 80 consecutive amino acids, and most preferably at least about 100 consecutive amino acids of the integrase polypeptide sequence.

The disclosure also provides analogs of the integrase or polypeptide. These analogs may differ from the natural integrase polypeptide in amino acid sequence and/or in modification that does not affect the sequence. These polypeptides include natural or induced genetic variants. Induced variants may be obtained through various techniques, such as random mutagenesis generated by radiation or exposure to mutagens, or through site-directed mutagenesis or other known molecular biology techniques. Analogs also include those with residues different from natural L-amino acids (e.g., D-amino acids) and those with non-naturally occurring or synthetic amino acids (e.g., β or γ-amino acids). It should be understood that the polypeptides of the disclosure are not limited to the representative polypeptides exemplified above.

Modified forms, which usually do not alter the primary structure, includes: chemically modified form of the polypeptide *in vivo* or *in vitro,* such as acetylation or carboxylation. Modification also includes glycosylation, such as those produced by glycosylation modification during polypeptide synthesis and processing or in further processing steps. Such modifications may be accomplished by exposing the polypeptide to glycosylation enzymes (e.g., mammalian glycosylases or deglycosylases). Modified forms also include sequences with phosphorylated amino acid residues (e.g., phosphotyrosine, phosphoserine, phosphothreonine). They also include polypeptides modified to improve their anti-proteolytic properties or optimize their solubility.

In the disclosure, the term "conservative variant" of integrase polypeptide refers to a polypeptide formed by replacing at most 10, preferably at most 8, more preferably at most 5, and most preferably at most 3 amino acids in the amino acid sequence represented by any one of SEQ ID NOs: 1-7 with amino acids having similar or analogous properties thereto. These conservative variant polypeptides are preferably generated by amino acid substitutions shown in Table 1.

**Table 1**

| Original Residue | Representative Substitutions | Preferred Substitution |
|---|---|---|
| Ala(A) | Val; Leu; Ile | Val |
| Arg(R) | Lys; Gln; Asn | Lys |
| Asn(N) | Gln; His; Lys; Arg | Gln |
| Asp(D) | Glu | Glu |
| Cys(C) | Ser | Ser |
| Gln(Q) | Asn | Asn |
| Glu(E) | Asp | Asp |
| Gly(G) | Pro; Ala | Ala |
| His(H) | Asn; Gln; Lys; Arg | Arg |
| Ile(I) | Leu; Val; Met; Ala; Phe | Leu |
| Leu(L) | Ile; Val; Met; Ala; Phe | Ile |
| Lys(K) | Arg; Gln; Asn | Arg |
| Met(M) | Leu; Phe; Ile | Leu |
| Phe(F) | Leu; Val; Ile; Ala; Tyr | Leu |
| Pro(P) | Ala | Ala |
| Ser(S) | Thr | Thr |
| Thr(T) | Ser | Ser |
| Trp(W) | Tyr; Phe | Tyr |
| Tyr(Y) | Trp; Phe; Thr; Ser | Phe |
| Val(V) | Ile; Leu; Met; Phe; Ala | Leu |

The polynucleotides of the disclosure can be in the form of DNA or RNA. DNA includes cDNA, genomic DNA, or artificially synthesized DNA. DNA may be single-stranded or double-stranded. DNA may be a coding strand or a non-coding strand. The coding region sequence encoding the mature polypeptide may be identical to the coding region sequence shown in any one of SEQ ID NOs: 10-16 or a degenerate variant. As used herein, taking integrase Cyin14 as an example, a "degenerate variant" in the disclosure refers to a nucleic acid sequence that encodes the protein of SEQ ID NO: 1 but differs from the coding region sequence of SEQ ID NO: 10.

Polynucleotides encoding the mature polypeptide include: a coding sequence encoding only the mature polypeptide; a coding sequence of the mature polypeptide and various additional coding sequences; a coding sequence of the mature polypeptide (and optional additional coding sequences) and non-coding sequences.

The term "polynucleotide encoding a polypeptide" may include a polynucleotide encoding the polypeptide, or include a polynucleotide further including additional coding and/or non-coding sequences.

The disclosure also relates to variants of the above polynucleotides, which encode polypeptides or fragments, analogs, and derivatives of polypeptides having the same amino acid sequence as those of the disclosure. Variants of such polynucleotides may be naturally occurring allelic variants or non-naturally occurring variants. These nucleotide variants include substitution variants, deletion variants, and insertion variants. As known in the art, an allelic variant is an alternative form of a polynucleotide, which may involve substitution, deletion, or insertion of one or more nucleotides, but does not substantially alter the function of the polypeptide it encodes.

The disclosure also relates to polynucleotides that hybridize with the above sequences and have at least 50%, preferably at least 70%, more preferably at least 80% identity between the two sequences. The disclosure particularly relates to polynucleotides that can hybridize with the polynucleotides of the disclosure under stringent conditions. In the disclosure, "stringent conditions" refer to: (1) hybridization and elution at low ionic strength and a high temperature, such as 0.2×SSC, 0.1%SDS, 60°C; or (2) presence of a denaturant during hybridization, such as 50% (v/v) formamide, 0.1% calf serum/0.1% Ficoll, 42°C, etc.; or (3) hybridization occurring only when the identity between the two sequences is at least 90% or more, more preferably 95% or more. Moreover, the polypeptide encoded by a hybridizable polynucleotide has the same biological function and activity as the mature polypeptide represented by SEQ ID NO: 2.

The disclosure also relates to nucleic acid fragments that hybridize with the above sequences. As used herein, a "nucleic acid fragment" contains at least 15 nucleotides in length, preferably at least 30 nucleotides, more preferably at least 50 nucleotides, and most preferably at least 100 nucleotides or more. Nucleic acid fragments may be used in nucleic acid amplification techniques (such as PCR) to identify and/or isolate polynucleotides encoding integrases.

The polypeptides and polynucleotides of the disclosure are preferably provided in an isolated form, and more preferably purified to homogeneity.

The full-length nucleotide sequence of the integrase of the disclosure or fragments thereof may usually be obtained by PCR amplification, recombination, or artificial synthesis. For PCR amplification, primers can be designed based on the relevant nucleotide sequences disclosed in the disclosure, especially the open reading frame sequence, and a commercially available cDNA library or a cDNA library prepared by conventional methods known to those skilled in the art may be used as a template for amplification to obtain the relevant sequence. When the sequence is long, it is often necessary to perform two or more PCR amplifications, and then splice the fragments amplified in each amplification in the correct order.

Once a relevant sequence is obtained, recombinant methods can be used to obtain the relevant sequence in large quantities. This is usually done by cloning it into a vector, then transferring it into cells, and then isolating the relevant sequence from the proliferated host cells by conventional methods.

In addition, artificial synthesis can also be used to synthesize the relevant sequences, especially when the fragment length is short. Usually, a very long fragment can be obtained by first synthesizing multiple small fragments and then ligating them.

At present, the DNA sequence encoding the protein of the disclosure (or its fragments, or its derivatives) can be obtained entirely by chemical synthesis. This DNA sequence can then be introduced into various existing DNA molecules (such as vectors) and cells known in the art. In addition, mutations can be introduced into the protein sequence of the disclosure by chemical synthesis.

The method for amplifying a DNA/RNA using the PCR technology (Saiki, et al. Science 1985; 230:1350-1354) is preferably used to obtain the gene of the disclosure. Especially when it is difficult to obtain a full-length cDNA from a library, the RACE method (RACE-rapid amplification of cDNA ends) can be preferably used. Primers for PCR can be appropriately selected based on the sequence information of the invention disclosed herein and can be synthesized by conventional methods. Amplified DNA/RNA fragments can be isolated and purified by conventional methods such as gel electrophoresis.

The disclosure also relates to vectors containing the polynucleotides of the disclosure, host cells genetically engineered with the vectors or integrase-encoding sequences of the disclosure, and methods for producing the polypeptides of the disclosure by recombinant technology.

The polynucleotide sequences of the disclosure can be used to express or produce recombinant integrase polypeptides by conventional recombinant DNA technology (Science, 1984; 224: 1431). Generally, the steps are as follows:
(1) Transforming or transducing a suitable host cell with the polynucleotide (or a variant) encoding the integrase polypeptide of the disclosure or with a recombinant expression vector containing the polynucleotide;
(2) Culturing the host cell in a suitable medium;
(3) Isolating and purifying the protein from the culture medium or cells.

In the disclosure, the integrase polynucleotide sequence can be inserted into a recombinant expression vector. The term "recombinant expression vector" refers to bacterial plasmids, phages, yeast plasmids, plant cell viruses, mammalian cell viruses such as adenoviruses, retroviruses, or other vectors known in the art. Vectors suitable for the disclosure include, but are not limited to: T7-based expression vectors for expression in bacteria (Rosenberg, et al. Gene, 1987, 56:125); pMSXND expression vectors for expression in mammalian cells (Lee and Nathans, J Bio Chem. 263: 3521, 1988); and baculovirus-derived vectors for expression in insect cells. In short, any plasmids and vectors may be used as long as they can replicate and be stable in a host. An important feature of expression vectors is that they usually contain an origin of replication, a promoter, a marker gene, and a translation control element.

Methods well known to those skilled in the art may be used to construct expression vectors containing the integrase-encoding DNA sequence and appropriate transcription/translation control signals. These methods include *in vitro* recombinant DNA technology, DNA synthesis technology, in *vivo* recombination technology, etc. (Sambrook, et al. Molecular Cloning, a Laboratory Manual, Cold Spring Harbor Laboratory, New York, 1989). The DNA sequence can be effectively linked to an appropriate promoter in the expression vector to direct mRNA synthesis. Representative examples of these promoters are: the lac or *trp* promoter from *Escherichia coli;* the λ phage PL promoter; eukaryotic promoters including the CMV immediate early promoter, HSV thymidine kinase promoter, early and late SV40 promoters, retroviral LTRs, and other known promoters that can control gene expression in prokaryotic or eukaryotic cells or their viruses. The expression vector also includes a ribosome binding site for translation initiation and a transcription terminator.

In addition, the expression vector preferably contains one or more selectable marker genes to provide phenotypic traits for selecting transformed host cells, such as dihydrofolate reductase, neomycin resistance, and green fluorescent protein (GFP) for eukaryotic cell culture, or tetracycline or ampicillin resistance for *Escherichia coli.*

Vectors containing the appropriate DNA sequences described above, as well as appropriate promoters or control sequences, can be used to transform appropriate host cells to enable them to express the protein.

Host cells can be prokaryotic cells, such as bacterial cells; or lower eukaryotic cells, such as yeast cells; or higher eukaryotic cells, such as mammalian cells. Representative examples include:
*Escherichia coli,* Streptomyces; bacterial cells of *Salmonella typhimurium;* fungal cells such as yeast; plant cells; insect cells of Drosophila S2 or Sf9; animal cells such as CHO, COS, 293 cells, or Bowes melanoma cells.

When the polynucleotide of the disclosure is expressed in higher eukaryotic cells, transcription will be enhanced if an enhancer sequence is inserted into the vector. Enhancers are cis-acting elements of DNA, usually of about 10 to 300 base pairs, which act on promoters to enhance gene transcription. Examples include the 100 to 270 bp SV40 enhancer at the late side of the replication origin, the polyoma enhancer at the late side of the replication origin, and adenovirus enhancers.

Those of ordinary skill in the art know how to select appropriate vectors, promoters, enhancers, and host cells.

Transformation of host cells with recombinant DNA may be performed by conventional techniques well known to those skilled in the art. When the host is a prokaryote such as Escherichia coli, competent cells capable of taking up DNA may be harvested after the exponential growth phase and treated by the CaCl₂ method, the steps of which are well known in the art. Another method is to use MgCl₂. If necessary, transformation can also be performed by electroporation. When the host is a eukaryote, the following DNA transfection methods can be selected: calcium phosphate co-precipitation, conventional mechanical methods such as microinjection, electroporation, liposome packaging, etc.

The obtained transformants can be cultured by conventional methods to express the polypeptide encoded by the gene of the disclosure. Depending on the host cell used, the medium used for culturing may be selected from various conventional media. Culturing is carried out under conditions suitable for the growth of the host cell. When the host cell grows to an appropriate cell density, the selected promoter is induced by a suitable method (such as temperature shift or chemical induction), and the cell is cultured for a further period of time.

The recombinant polypeptide in the above method can be expressed in a cell or on the cell membrane, or secreted extracellularly. If needed, the recombinant protein can be isolated and purified by various separation methods using its physical, chemical, and other properties. These methods are well known to those skilled in the art. Examples of these methods include, but are not limited to: conventional renaturation treatment, treatment with protein precipitants (salting-out method), centrifugation, osmotic lysis, ultra sonication treatment, ultracentrifugation, molecular sieve chromatography (gel filtration), adsorption chromatography, ion exchange chromatography, high-performance liquid chromatography (HPLC), and various other liquid chromatography techniques and combinations of these methods.

Recombinant integrases or polypeptides have various uses. These uses include (but are not limited to): integrating target sequences into mammalian genomes, thereby treating certain diseases or conferring certain corresponding functions.

### Reaction Method and System for Integration

The disclosure provides a gene integration method, the method comprising:
(a) providing a target nucleic acid to be integrated and a host genome;
(b) in the presence of the integrase of the disclosure, allowing the target nucleic acid to be integrated into the genome.

The disclosure also provides a reaction system for gene integration, the reaction system comprising:
(a) a target nucleic acid to be integrated;
(b) a host genome; and
(c) the integrase of the disclosure.

Preferably, the integration method may be an in vivo integration method or an in vitro integration method. In addition, the genome may be a nucleic acid molecule located in a prokaryotic cell, a eukaryotic cell, or isolated from a large-fragment genome.

Preferably, the host cell includes a mammalian cell, especially human and non-human mammalian cell; the cell may be a somatic cell or germ cell.

### Main Advantages of the Disclosure

1. The integrases of the disclosure have low homology with previously reported integrases, exhibit excellent integration activity, and have a bright prospect of wide applications.
2. These integrases of the disclosure can be used to integrate large DNA fragments encoding therapeutic genes into a genome without relying on viral vectors, thereby greatly improving the safety of gene therapy, such as the DNA fragments of CAR molecules in CAR-T therapy, coagulation factor-encoding genes in hemophilia gene therapy, etc.
3. Combined with PE (Prime Editor) technology, it can realize insertion of integration sequences at specific genomic positions, achieving site-specific integration of large-fragment DNA.

The disclosure is further illustrated below with specific examples. It should be understood that these examples are only for illustrating the disclosure and not for limiting the scope of the disclosure. The experimental methods without specific conditions indicated in the following examples generally follow conventional conditions, such as those described in Sambrook et al., Molecular Cloning: A Laboratory Manual (New York: Cold Spring Harbor Laboratory Press, 1989), or according to the conditions recommended by the manufacturer. Unless otherwise specified, percentages and parts are by weight.

### Example 1. Identification of Integrases

### 1. Acquisition of Integrases

The inventors identified multiple novel integrases through mining metagenomic data using computational programs, analyzing metagenomes of uncultured organisms, and performing de-redundancy and protein clustering analyses. These integrases were named Cyin10, Cyin11, Cyin12, Cyin13, Cyin14, Cyin15, and Cyin16, and their amino acid sequences are shown in SEQ ID No. 1-7, respectively.

The amino acid sequences of the integrases are as follows:
Cyin14 
MAKAAGYVRLSKYDDATTSLARQREAIEKLCADRGWELAETFEDVDRSGWNGVHRPGLDRMLASLDGVGAL
VVYRIDRLARSQADFARILETLRDAGVDFVATDLQVDSSPAGTLVRDLVARLAQFESDQISVRSKAMMAYKAKK
GEPVGRVPYGWRRNGKTYEPDPEQQAALREAAARFIAGETFSAIAPGVGLPVASLSRILRSQRVLDALPPELSGP
LAQALRDRRWQRVPTSKQSLLGGIARCAECGTTMTKVSTRGGRKNGRWYSYACRDSHAAISGQWLDAYVTRE
VLDAADTGRLLTALKKRRKVKTPRRVSEIEARLELLEHDHYVTGKIPAARFERLRDALLDQLKAAQKVERDADVDL
PAELARDLGERWGDLSIGTRRRIVRACLEGIEVEKAKGHGRVDPGRVRLVWRVWR*(SEQ ID No.1)
Cyin10 
MARNITVIPARANRTNLAQNLEPQKKKMAAYCRVSTDQLEQLSSYEVQVNYYTNYINNSSEYEMVNVYADEGI
SGTNTKKREQFNKMIDDCKAGKIDIIITKSISRFARNTLDTLNYVRTLKELGIGVIFEKENINTLDSKGEVLLTILSSLA
QDESRSISENSTWGIRRKFEQGKVSVNCTKFLGYDKGEDGNLVINEKQAKIVRRIYKDYLEGKGTNRIARELEEEG
IPNWNGKAKWYESSIRNMLSNEKYKGEALLQKTYTVNFLTKKRVINNGEIPQYYVEESHPAIINKEIWEAVQLEM
ERRKAFAEKYNISKLDYATVDNPFAGRVVCGHCGSTFGRKVWNSTNERNRRVIWRCNRKYEVKGKKSCENKHI
DDRVL*(SEQ ID No.2)
Cyin11 
MMEEKKILRCAIYTRKSTEEGLDMDYNTLDAQREAGEKYVEAMRGEGWRIIPDLYDDGGFSGGNMERPALKR
LIDDIKAGRIDIVVVYKVDRLSRSLMDFAKLVEILDKHGVCFVSVTQHFNTKDSMGRLTLNILLSFAQFEREVTGER
IRDKFASSKMKGMWMGGPPPLGYDVVDRKLVTNPKEVELVNIIFDRFAALGSIALLAQELNKAGHRTKHYISRT
GKPVGGKPFTENPLRTFLQNRLFIGEVHHKGKYYPGQHQPIITQAQFDAAQTIFAESSKGKRKRTFQAKSKALLK
GILVCGGCGGAMSPTHTNKGKKNYHYYSANSYRKQSCELCPANRIPAGEIEEVVKGQLQSIFVRPQVIVDVWKK
VKTDIPNYNEIDLHSTMQRMGSLWDILVPAEQKRITHLLIDRVAVHGNCIDIEYRANGIEDILSQLQQEIEQKVKN
GAEA*(SEQ ID No.3)
Cyin12 
MKTSKAILYVRVSTDEQAEKGHSLAHQEERLRNYCKTNNIEVVAFFKDDHSAKSFDRPEFKKLLAFIKTNKHLAD
MLLFLKWDRFSRNAADAYAMINQLTRLGVDPQAMEQPLNMEIPEHKFMLAIYLAAPEVENDRRALNIIAGMR
KAMKEGRWMGSAPKGYKRSHDENNRPCIVPGKDVEMVRWIFEQMATGVYHIDELRKIVNSKGFKIERNGFW
WLLRSPVYIGKVSVAAYKDEPAQVVQGKHEPIISENLFYAVQEVLEGRKRIDLPPQYSKRDELPLRGFLKCPNCGG
NLTGSGSKGNGGRYFYYHCKSKCKIRYRATEVNQGFDDELNFISECEKVLRSFDRMLSNHRKENQKDKSSELTKL
KKDLENCDKRLENAQTLMLDEKLEANEYKNIKSRLEPEKGKLLMKISQLEEKDPEENKIKEFGFNYLRNLNQLFWI
ATLEDRHTLLCSTLSDKLKFEDGKVRTMSGNDIISLIRKADKRLEVNKKGQLKNFSCPSHRVETTGIEPVSKHILQK
LSTCLFCFGFSGSNWKQTTDHFLSCIVFCYSHSLLQQHPVLFVIGRGSGQQDCLPPGHNDYLITD*(SEQ ID
No.4)
Cyin13 
MKQQKAILYIRVSTDEQADKGYSLKHQEERLRSYCQFQNIEVIGLYKDDHSAKTFERPAFKQLLSFVKKNRKSSNL
LLFTKWDRFSRNAGDAYAMINTLNGYGIEPQAVDQPLDLNVPENKIMLAFYLAAPEVENDRRALNVIVGMRRA
KKDGRWMATAPKGYKNIRTDQNRPIIVPSPDAKYIKWVFEEINKGALHADQIRKIANKKGFEISRGYFWTMIRN
PVYCGKIFIPAYKEEDACYVQGIHEPLISEELFYNVQDILNGRKKNVQNRSAQKKELPLRGFLICKKCGGNLTGSAS
KGTGGKYFYYHCQPGCNERIKAEEANKKVINELKKIPAKKEAIDLYRIILEKQLKKNSKESISDKEKIETEIKKNKERID
 
NAQQLMLDGELPMSEYKVIKSRYDRIIKELQEQMTNSSSTVSGYKKYLDFGFNFLQTVDQFYVSAKPAIQLQTLS
SIYAEKLVFENNSYRTPVYKEELSLILNTGRSFGGNKKGQNQKNLILSSGVAPTGIEPVSKV*(SEQ ID No.5)
Cyin15 
MNTATAVQEQVLVYLRISEDRTGEEAGVERQRADCFELCERLGFLTPQVFVDNDVSAFKGTRRPGYQALLERVV
TGPARVVVWHVDRLYRRPRELEDLIDLVESSPIRLEAVKGGAFDLNTHEGRLMARQLVAIASYESGHKADRIRRA
NQQKAERGDWHGAPRFGYGPGGVVVPREAMIVREMADRFLAGHTLRAITRWLNDESGVAPPMARKGTSGV
WHATTVKSILSSARISGQRAYDPDREGREPPEGRAILGTGNWQPIITPAETARIRAMLGAPDRRTSASSIRLLSAI
ATCGRCGAGLVSGTNPRARDQAHRRRYICKRIYGIPERGGLSVSAYPVERLVEEAVITRLANTKLIQPTSDEGDPE
ARAAVDAVVEARQRIEDLAREYGAGRLTRQEFQLARGAANASIEEAERALARTAKTNALSGLPVGDEIALRAAW
SEWLIPQRRAILSALIDSLVVRPQPPRGPRFNPDRVELRFKV*(SEQ ID No.6)
Cyin16

MRAFGYVRISKLDADTTSPQRQREAIERLCADRGWELVRVFEDLDLSAYRRDVKRPGFEAMMARLGEIDRIVF
WRLDRLARSVATFSRALEACREAAVELVSTDHQIDTGSAMGRAFVQITATFAELESGTLSERARQMHAHLRAK
GQPVGRVPFGWRRNGKGLEPVPEQQAVLVEAARRYVAGESLRSIAAGVGIHHPNLAQRLRSDRVIEALPPELG
ARLVETLAERGRTGTRAKQSLLGGIARCAVCGAGLTIVGQRMGNGDRKAWGAYACREKGHVTISRGWLDDHI
TAEVVAAIDTGKLVERLAKRKRTRRTRPVSEIEARLELLERDFYERGMLSRDSFLRRREGLLRRLAEARASEENAGI
DLPRELAMNLGAAWPTLSMHGRRRIISAVIARVEVSKASNHGRIDPSRVRLAWRE*(SEQ ID No.7)

### 2. Optimization of Integrases

The inventors performed human codon optimization on the obtained integrases (Cyin10, Cyin11, Cyin12, Cyin13, Cyin14, Cyin15, Cyin16) for further functional experiments.

The codon-optimized coding sequences are SEQ ID NOs: 10-16, respectively, and the sequences are as follows:
Cyin14 
ATGGCCAAGGCCGCCGGATACGTGAGACTGTCCAAGTACGACGCCACCACCAGCCTGGCCCGGCAGA
GAGAGGCCATCGAGAAGCTGTGCGCCGACCGGGGATGGGAGCTGGCCGAGACATTTGAGGATGTGGACC
GCAGCGGCTGGAACGGCGTGCATAGGCCAGGCCTGGACCGGATGCTGGCCTCCCTGGATGGCGTGGGCG
CTCTGGTGGTGTACCGGATTGACAGACTGGCCAGATCCCAGGCCGACTTCGCCAGAATCCTGGAAACCCTG
AGGGACGCTGGCGTGGACTTCGTGGCCACCGACCTGCAGGTGGATAGCTCCCCCGCTGGCACCCTGGTGA
GGGATCTGGTGGCTCGGCTGGCCCAGTTCGAGAGCGACCAAATCTCCGTGAGGAGCAAGGCTATGATGGC
 
CTACAAAGCCAAGAAGGGCGAACCAGTGGGCAGAGTGCCCTACGGCTGGAGGAGAAATGGCAAGACCTA
CGAGCCCGACCCCGAACAGCAGGCCGCCCTGAGGGAGGCCGCCGCCAGATTCATCGCCGGAGAGACATTC
AGCGCCATCGCCCCAGGCGTGGGCCTGCCCGTGGCCTCCCTGTCCCGGATTCTGAGAAGCCAGCGGGTGC
TCGACGCCCTGCCACCCGAGCTGAGCGGCCCCCTGGCCCAGGCCCTGAGGGATAGAAGATGGCAGCGGG
TGCCTACCAGCAAGCAGTCTCTGCTGGGGGGCATCGCCAGATGTGCTGAATGTGGCACCACCATGACCAA
GGTGAGCACACGGGGCGGCAGAAAGAACGGCCGGTGGTACAGCTACGCCTGCAGAGACTCCCACGCCGC
CATCTCTGGCCAGTGGCTGGACGCCTACGTGACCAGGGAGGTGCTGGACGCCGCCGACACCGGGCGGCT
GCTGACAGCTCTGAAGAAGAGGAGGAAGGTCAAGACCCCTAGGCGGGTGAGCGAGATCGAGGCCCGGCT
GGAGCTGCTGGAGCACGACCACTACGTGACCGGAAAGATCCCCGCCGCCAGGTTTGAGAGGCTGCGCGAC
GCCCTGCTGGACCAACTGAAGGCCGCCCAGAAGGTGGAACGCGACGCCGATGTGGATCTGCCTGCCGAGC
TGGCCAGGGACCTGGGCGAGCGCTGGGGCGACCTGAGCATCGGCACCAGGAGGCGGATCGTGAGAGCCT
GCCTGGAGGGCATCGAGGTGGAGAAGGCCAAGGGCCACGGCAGAGTGGACCCCGGCAGAGTGAGACTG
GTGTGGAGAGTGTGGAGATGA(SEQ ID No.10)
Cyin10 
ATGGCCAGAAACATCACCGTGATCCCCGCCAGAGCCAACAGAACCAACCTGGCCCAGAACCTGGAGCCCC
AGAAGAAGAAGATGGCCGCCTACTGCAGAGTGAGCACCGACCAGCTTGAGCAGCTCAGCAGCTACGAGG
TGCAGGTGAACTACTACACCAACTACATCAACAACAGCAGCGAGTACGAGATGGTGAACGTGTACGCCGA
CGAGGGCATCTCCGGCACCAACACCAAGAAGCGCGAGCAGTTCAACAAGATGATCGACGACTGCAAGGCC
GGAAAGATCGACATCATCATCACCAAGAGCATCAGCAGATTCGCCAGAAACACCCTGGACACCCTGAACTA
CGTGAGAACCCTGAAGGAGCTGGGCATCGGCGTGATCTTCGAGAAGGAGAACATCAACACACTGGACAG
CAAGGGCGAGGTGCTGCTGACCATCCTGTCCAGCCTGGCTCAGGACGAGAGCAGGTCCATCAGCGAGAAC
AGCACCTGGGGCATCAGGAGAAAGTTCGAGCAGGGCAAGGTGAGCGTGAACTGCACCAAGTTCCTGGGA
TACGACAAGGGCGAGGACGGCAACCTCGTGATCAACGAGAAGCAGGCCAAGATCGTGAGGAGGATCTAC
AAGGACTACCTGGAGGGCAAGGGCACCAACAGAATCGCCAGAGAGCTGGAGGAGGGCATCCCTAAC
TGGAACGGCAAGGCCAAGTGGTACGAGTCCAGCATCAGGAACATGCTGTCTAATGAGAAGTATAAGGGC
GAGGCCCTGCTGCAGAAAACCTACACCGTGAACTTCCTGACCAAGAAGAGAGTGATCAATAATGGCGAGA
TCCCCCAGTACTACGTGGAGGAGTCCCACCCCGCCATCATCAACAAGGAGATTTGGGAGGCCGTGCAACTG
GAGATGGAGAGAAGAAAGGCCTTTGCCGAGAAGTACAACATCAGCAAGCTGGACTACGCCACCGTGGAC
AACCCCTTCGCCGGAAGAGTGGTGTGCGGCCACTGCGGCAGCACCTTCGGCAGAAAGGTGTGGAATAGCA
CCAACGAGAGAAACAGGAGAGTGATCTGGAGATGCAACAGGAAGTACGAGGTGAAGGGCAAGAAGAGC
TGCGAGAACAAGCACATCGACGACAGAGTGCTGTGA(SEQ ID No.11)
Cyin11 
ATGATGGAGGAGAAGAAGATCCTGAGATGCGCCATCTACACCAGGAAGTCCACCGAGGAGGGCCTGGAT
ATGGACTACAACACCCTGGACGCCCAGAGAGAGGCCGGAGAGAAGTACGTGGAGGCCATGCGGGGCGA
GGGCTGGAGGATCATTCCCGACCTGTACGACGACGGCGGCTTCAGCGGCGGCAACATGGAGCGGCCCGC
CCTGAAGAGGCTGATCGACGATATTAAGGCCGGAAGAATCGACATCGTGGTGGTCTACAAGGTGGACAGA
CTGAGCCGGAGCCTGATGGACTTCGCCAAGCTCGTGGAGATCCTGGACAAGCACGGCGTGTGCTTCGTGA
GCGTGACTCAGCACTTCAATACCAAGGACTCTATGGGCAGACTGACCCTGAACATCCTGCTGTCCTTCGCCC
AGTTCGAGAGAGAGGTGACAGGCGAGAGAATCAGAGACAAGTTCGCCAGCAGCAAGATGAAGGGCATGT
GGATGGGCGGCCCTCCCCCCCTGGGCTACGATGTGGTGGACAGAAAGCTGGTGACCAACCCTAAGGAGGT
GGAGCTGGTGAATATCATCTTCGACAGGTTTGCCGCCCTGGGCTCCATCGCCCTGCTGGCCCAGGAGCTGA
ATAAGGCTGGCCACAGGACCAAGCACTACATCAGCAGGACCGGCAAGCCCGTGGGCGGCAAGCCCTTCAC
CGAGAACCCTCTGAGAACCTTCCTGCAGAATAGACTGTTCATCGGGGAGGTGCACCACAAGGGCAAGTAC
TACCCTGGCCAGCACCAGCCCATCATCACCCAGGCCCAGTTCGATGCCGCCCAGACCATCTTCGCCGAGAG
CAGCAAGGGCAAGAGAAAGAGAACCTTCCAGGCCAAGAGCAAGGCCCTGCTGAAGGGCATCCTGGTGTG
CGGCGGCTGCGGCGGGGCCATGAGCCCCACCCACACCAACAAGGGCAAGAAGAACTACCACTACTACAGC
GCCAACAGCTACCGCAAGCAGAGCTGCGAGCTGTGCCCCGCCAACAGAATCCCCGCTGGCGAGATTGAGG
AGGTGGTGAAGGGCCAGCTCCAGAGCATCTTCGTGAGGCCTCAAGTGATCGTGGATGTGTGGAAGAAGG
TCAAGACCGACATCCCCAACTACAATGAGATCGACCTGCACAGCACCATGCAGAGAATGGGCTCCCTGTGG
GACATCCTGGTGCCTGCCGAGCAGAAGCGGATCACACACCTGCTGATCGACCGGGTGGCCGTGCACGGCA
ACTGCATCGACATCGAGTACAGGGCCAACGGCATCGAGGACATCCTGAGCCAGCTCCAGCAGGAGATCGA
GCAGAAGGTGAAGAACGGAGCCGAGGCCTGA(SEQ ID No.12)
Cyin12 
ATGAAAACCAGCAAGGCCATCCTGTACGTGAGGGTGAGCACAGACGAGCAGGCCGAGAAGGGCCACAGC
CTGGCCCACCAGGAGGAGCGCCTGAGGAACTACTGCAAGACCAACAACATCGAGGTGGTGGCCTTCTTCA
AGGACGACCACAGCGCCAAGAGCTTTGACAGGCCCGAGTTTAAGAAGCTGCTGGCCTTCATCAAGACAAA
CAAGCACCTGGCCGACATGCTGCTGTTTCTCAAGTGGGACAGATTCTCCAGGAACGCCGCCGATGCCTACG
CCATGATCAATCAGCTCACCCGGCTGGGCGTGGACCCACAGGCAATGGAGCAGCCCCTGAATATGGAGAT
CCCTGAGCATAAGTTTATGCTGGCCATCTATCTGGCCGCCCCAGAAGTGGAGAACGACCGGAGGGCACTG
AACATCATTGCTGGCATGAGGAAGGCCATGAAGGAGGGCAGATGGATGGGCTCCGCCCCAAAGGGCTAC
AAGAGATCCCACGATGAGAACAACAGGCCTTGCATCGTGCCTGGAAAAGATGTGGAGATGGTGAGATGG
ATCTTCGAGCAGATGGCCACCGGCGTGTACCACATCGACGAGCTGAGAAAGATCGTGAACTCCAAAGGCT
TTAAGATTGAGAGAAACGGGTTCTGGTGGCTGCTGAGAAGCCCCGTCTACATCGGGAAGGTGAGCGTGGC
CGCCTACAAGGACGAGCCCGCCCAGGTGGTGCAGGGCAAGCACGAGCCAATCATTTCCGAGAACCTGTTC
TACGCCGTGCAGGAAGTGCTGGAGGGCAGAAAGCGGATCGACCTGCCACCCCAGTATAGCAAGAGGGAC
 
GAACTGCCTCTGAGAGGCTTTCTGAAGTGTCCCAACTGCGGCGGCAACCTGACCGGGTCTGGCAGCAAGG
GCAACGGCGGCAGATACTTCTATTATCACTGCAAGAGCAAGTGCAAAATCAGGTATAGAGCCACCGAGGT
GAACCAGGGGTTTGACGACGAGCTGAATTTTATCAGCGAATGCGAGAAGGTGCTGCGGAGCTTCGACCGG
ATGCTGAGCAACCATCGCAAGGAGAACCAGAAGGACAAGTCTAGCGAGCTGACAAAGCTGAAGAAGGAT
CTGGAGAACTGTGACAAGCGGCTGGAGAACGCTCAGACACTGATGCTGGACGAGAAGCTGGAGGCCAAC
GAGTACAAGAATATCAAGAGCAGGCTGGAGCCAGAGAAGGGAAAGCTGCTGATGAAAATCTCCCAGCTT
GAGGAAAAAGACCCCGAAGAGAACAAGATCAAGGAGTTTGGCTTCAACTACCTGAGAAATCTGAACCAGC
TCTTCTGGATCGCCACCCTGGAGGACAGGCACACACTGCTGTGTAGCACTCTGTCCGACAAGCTGAAGTTT
GAGGACGGCAAGGTGAGAACAATGAGCGGAAACGACATCATCTCTCTGATTCGGAAGGCCGATAAGAGA
CTGGAGGTGAACAAGAAGGGCCAGCTCAAGAACTTCTCCTGCCCCTCCCACAGGGTGGAGACAACCGGCA
TTGAGCCCGTGTCCAAGCACATCCTGCAGAAGCTGAGCACCTGCCTGTTCTGTTTCGGCTTCAGCGGCTCTA
ATTGGAAGCAGACCACCGACCACTTCCTGAGCTGTATCGTGTTCTGCTACTCCCACTCCCTGCTGCAGCAGC
ACCCTGTGCTGTTCGTGATTGGCAGGGGCAGCGGCCAGCAGGATTGCCTGCCTCCTGGCCACAACGACTAC
CTGATCACTGACTGA(SEQ ID No.13)
Cyin13 
ATGAAGCAGCAGAAGGCCATCCTGTATATCAGAGTGAGCACCGACGAGCAGGCCGACAAGGGCTACAGC
CTGAAGCACCAGGAGGAGACTGAGAAGCTACTGCCAGTTCCAGAACATTGAAGTGATCGGCCTGTATA
AGGACGACCACAGCGCCAAGACATTCGAGAGGCCCGCCTTCAAGCAGCTCCTGAGCTTCGTGAAGAAGAA
TAGGAAGAGCAGCAATCTGCTGCTGTTTACCAAGTGGGACAGGTTCAGCAGAAACGCCGGTGACGCCTAC
GCCATGATCAATACCCTGAATGGCTACGGGATCGAGCCTCAGGCCGTGGACCAGCCCCTGGACCTGAACG
TGCCCGAGAATAAGATCATGCTGGCCTTTTACCTGGCCGCTCCCGAGGTGGAGAATGACAGAAGGGCTCT
GAACGTGATCGTGGGCATGAGAAGAGCCAAAAAGGATGGCAGATGGATGGCCACCGCCCCTAAGGGCTA
CAAGAACATTAGGACAGACCAGAACAGACCAATCATCGTGCCTAGCCCCGACGCCAAGTACATCAAGTGG
GTGTTCGAGGAAATCAACAAGGGCGCTCTGCACGCCGACCAGATCAGAAAGATCGCCAACAAGAAGGGCT
TCGAGATTTCCCGCGGCTACTTCTGGACCATGATCAGGAACCCCGTGTACTGCGGCAAAATTTTCATCCCTG
CCTATAAGGAGGAGGACGCCTGCTACGTGCAGGGCATCCACGAGCCCCTGATCAGCGAAGAGCTGTTTTA
CAACGTGCAGGATATTCTGAATGGCAGAAAGAAGAACGTGCAGAACAGGAGCGCCCAGAAGAAGGAGCT
GCCACTGCGGGGCTTCCTGATCTGCAAGAAGTGCGGCGGCAACCTGACAGGCAGCGCCAGCAAGGGCAC
CGGCGGCAAGTACTTCTATTACCACTGCCAGCCCGGCTGCAATGAGAGAATCAAGGCCGAGGAGGCTAAT
AAGAAAGTGATCAACGAGCTGAAGAAGATCCCTGCCAAGAAGGAGGCCATCGACCTGTACCGCATCATCC
TGGAGAAGCAGCTCAAGAAGAACTCCAAGGAGTCCATCTCTGACAAGGAGAAGATCGAAACCGAGATCAA
GAAGAATAAGGAGAGAATCGACAACGCCCAGCAGCTCATGCTGGATGGCGAGCTGCCCATGAGCGAGTA
CAAAGTGATCAAGAGCAGATATGACAGAATCATCAAGGAGCTGCAGGAGCAGATGACCAACAGCAGCTCC
 
ACCGTGTCCGGCTACAAGAAGTACCTGGACTTCGGCTTCAACTTTCTGCAGACCGTGGACCAGTTCTACGT
GAGCGCCAAGCCTGCCATCCAGCTCCAGACCCTGTCCAGCATCTACGCCGAGAAGCTGGTGTTCGAGAACA
ATAGCTACCGGACCCCCGTCTACAAGGAGGAGCTGAGCCTGATCCTGAACACCGGCCGCAGCTTCGGCGG
CAATAAGAAGGGCCAGAACCAGAAGAATCTGATCCTGAGCAGCGGCGTGGCCCCCACCGGCATCGAGCCC
GTGAGCAAGGTGTGA(SEQ ID No.14)
Cyin15 
ATGAATACCGCCACCGCCGTGCAGGAGCAGGTGCTGGTGTACCTGCGGATCTCTGAGGACCGGACCGGCG
AGGAGGCCGGAGTGGAGAGACAGAGAGCCGACTGCTTCGAGCTGTGTGAGAGACTGGGCTTCCTGACAC
CCCAGGTGTTCGTGGACAACGATGTGAGCGCATTCAAGGGCACAAGGAGGCCAGGCTATCAGGCCCTGCT
GGAGAGGGTGGTGACTGGCCCCGCCAGGGTGGTGGTGGCACGTAGACCGCCTGTACCGCAGACCCAG
AGAGCTGGAGGATCTGATCGACCTCGTGGAGAGCAGCCCCATCCGGCTGGAGGCCGTGAAGGGCGGCGC
TTTCGACCTGAACACTCACGAGGGAAGGCTGATGGCCAGGCAACTGGTGGCCATCGCCAGCTACGAGTCT
GGCCACAAGGCTGACCGCATCAGAAGGGCCAACCAGCAGAAGGCCGAGCGCGGGGACTGGCACGGCGCT
CCTCGCTTCGGCTACGGCCCAGGCGGCGTGGTGGTGCCACGGGAGGCCATGATTGTGAGAGAGATGGCC
GATAGGTTCCTGGCCGGGCACACCCTGAGAGCCATTACCCGGTGGCTGAATGACGAGTCCGGCGTGGCCC
CTCCAATGGCCAGAAAGGGCACCTCTGGCGTGTGGCACGCCACCACCGTCAAATCTATCCTCAGCTCCGCC
AGGATCAGCGGCCAGAGAGCCTACGACCCAGACAGAGAGGGCAGGGAGCCCCCGAGGGCAGGGCCAT
CCTGGGCACCGGCAACTGGCAGCCCATTATCACCCCCGCCGAGACAGCCCGCATTAGGGCCATGCTGGGC
GCACCTGACAGACGGACCTCCGCCAGCAGCATCCGGCTGCTGAGCGCCATCGCCACCTGCGGAAGGTGCG
GCGCAGGGCTGGTGAGCGGCACCAACCCCCGCGCCCGCGATCAGGCCCACAGGCGGCGGTATATCTGCAA
GCGCATCTACGGCATCCCTGAGCGGGGCGGCCTGTCCGTGAGCGCCTACCCTGTGGAGAGACTGGTGGAG
GAGGCCGTGATCACCCGCCTGGCTAATACCAAGCTGATCCAGCCAACATCTGATGAGGGCGATCCTGAGG
CCAGAGCCGCTGTGGACGCCGTGGTGGAAGCCAGACAGCGGATCGAGGATCTGGCCAGGGAGTACGGCG
CTGGCAGACTGACAAGGCAGGAGTTTCAGCTCGCCAGAGGAGCCGCCAACGCCAGCATCGAGGAGGCCG
AGAGAGCCCTGGCCAGAACCGCCAAGACCAACGCCCTGAGCGGCCTGCCAGTGGGCGATGAGATCGCCCT
GAGGGCCGCCTGGTCTGAGTGGCTGATCCCACAGGAGAGCCATCCTGTCAGCCCTGATTGACAGCCTG
GTGGTGCGGCCTCAGCCCCCAGGGGACCCAGGTTTAACCCTGACAGAGTGGAGCTGCGGTTCAAGGTGT
GA(SEQ ID No.15)
Cyin16

ATGAGAGCCTTCGGCTACGTGAGAATCTCCAAGCTGGACGCCGACACCACCTCCCCCAGCGGCAGAGAG
AGGCCATCGAGAGACTGTGCGCCGACAGAGGCTGGGAGCTGGTGCGGGTGTTCGAGGACCTGGACCTGA
GCGCCTACCGGCGGGATGTGAAGAGGCCCGGCTTCGAGGCCATGATGGCTAGACTGGGCGAGATCGACA

GGATCGTGTTTTGGAGACTGGACCGGCTGGCCAGATCCGTGGCCACCTTCAGCAGAGCCCTGGAGGCCTG
CCGGGAGGCCGCCGTGGAGCTGGTGAGCACCGACCACCAGATCGACACAGGCTCCGCTATGGGCAGAGC
CTTCGTGCAGATCACCGCCACCTTCGCCGAGCTGGAGAGCGGCACCCTGAGCGAGAGGGCCAGGCAGATG
CACGCCCACCTGAGAGCCAAGGGCCAGCCCGTGGGGAGGGTGCCTTTCGGGTGGAGAAGAAACGGCAAG
GGCCTGGAGCCCGTGCCCGAGCAGCAGGCCGTGCTGGTGGAGGCCGCCAGACGGTATGTGGCCGGAGAG
AGCCTGAGGTCCATCGCCGCAGGCGTGGGCATCCACCACCCCAATCTGGCCCAGCGGCTGAGGAGCGACA
GGGTCATCGAGGCCCTGCCACCCGAGCTGGGAGCCAGACTGGTGGAGACACTGGCCGAGCGCGGCAGGA
CCGGCACCAGAGCCAAGCAGAGCCTGCTGGGCGGCATCGCCCGCTGCGCCGTGTGCGGCGCTGGCCTGAC
CATCGTGGGCCAGAGAATGGGCAACGGCGACAGAAAGGCCTGGGGAGCCTACGCCTGTCGGGAGAAGG
GCCATGTGACAATCAGCAGGGGCTGGCTGGATGACCACATCACCGCCGAGGTGGTGGCCGCCATCGACAC
CGGCAAGCTGGTGGAGCGCCTGGCCAAGAGAAAGAGAACCCGGCGGACCAGACCTGTGAGCGAGATCGA
GGCCAGACTGGAACTGCTGGAGAGAGATTTTTACGAGAGAGGCATGCTGAGCCGCGACAGCTTCCTGCGG
AGGCGGGAGGGCCTGCTGCGCAGACTGGCCGAGGCCAGAGCCAGCGAGGAGAACGCCGGAATCGACCT
GCCTCGCGAGCTGGCCATGAATCTGGGCGCTGCCTGGCCCACCCTGAGCATGCACGGCAGGAGGCGCATC
ATCAGCGCCGTGATCGCCAGAGTGGAGGTGTCCAAGGCCAGCAACCACGGCAGGATCGACCCTTCCAGGG
TGAGACTGGCCTGGAGAGAGTGA(SEQ ID No.16)

### 3. Analysis and Prediction of Domain Structures for Each Enzyme via Multiple Sequence Alignment Software

The online tool "Conserved Domain Search Service" in the "domain & structure" section of the NCBI website was used to predict the domains of integrases Cyin10 to Cyin16.

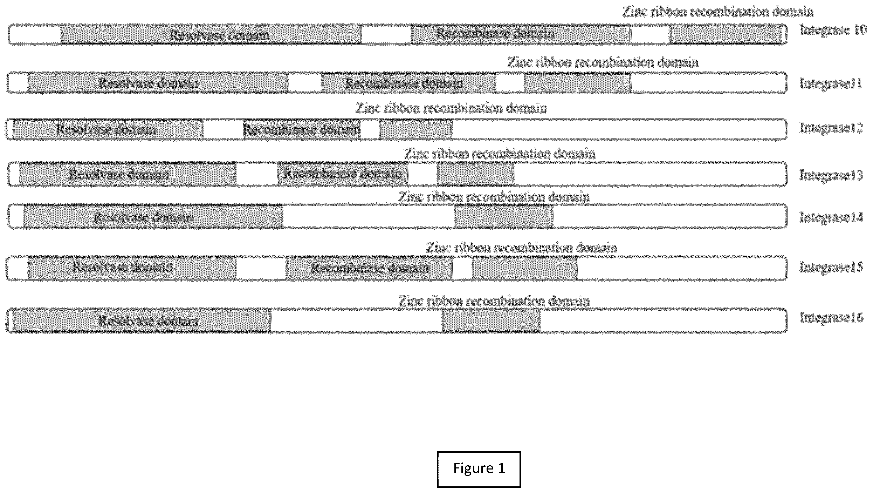

Analysis of the positions of functional domains of integrases Cyin10, Cyin11, Cyin12, Cyin13, Cyin14, Cyin15, and Cyin16 relative to the N-terminus and C-terminus of the proteins revealed that the distribution of each functional domain is as shown in Figure 1.

As can be seen from Figure 1, integrases 10-13 and 15 sequentially include, from N-terminus to C-terminus, a resolvase domain (Resolvase), a recombinase domain (Recombinase), and a zinc ribbon recombination domain (Zn_ribbon_recom). Integrases Cyin14 and Cyin16 only have a resolvase domain (Resolvase) and a zinc ribbon recombination domain (Zn_ribbon_recom). The predicted distribution of each integrase domain (calculated from N-terminus to C-terminus) is as follows:

**Table 1**

| Recombinase Name | Resolvase Domain | Recombinase Domain | Zn_ribbon_recom Domain |
|---|---|---|---|
| Cyin10 | 29-176 | 199-307 | 327-382 |
| Cyin11 | 10-163 | 183-279 | 296-357 |
| Cyin12 | 6-155 | 181-266 | 282-335 |
| Cyin13 | 6-155 | 181-266 | 281-334 |
| Cyin14 | 4-148 | None | 240-294 |
| Cyin15 | 13-149 | 175-277 | 291-355 |
| Cyin16 | 3-147 | None | 240-295 |

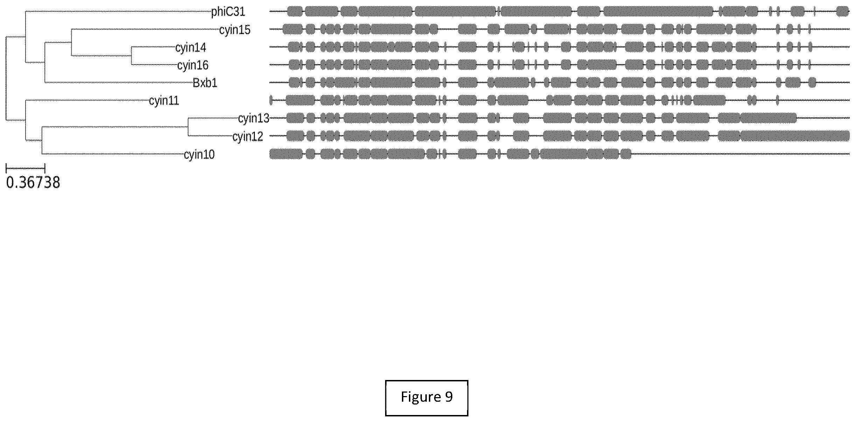

Sequence alignment analysis of the phylogenetics among integrases Cyin11-16 is shown in Figure 9. As can be seen from Figure 9, integrases Cyin12 and 13 are more closely related, and integrases Cyin14 and 16 are more closely related. Integrases Cyin10-13 belong to one large protein family, while integrases Cyin14-16 belong to another large protein family. This is also supported by the predictive analysis of integrase domains.

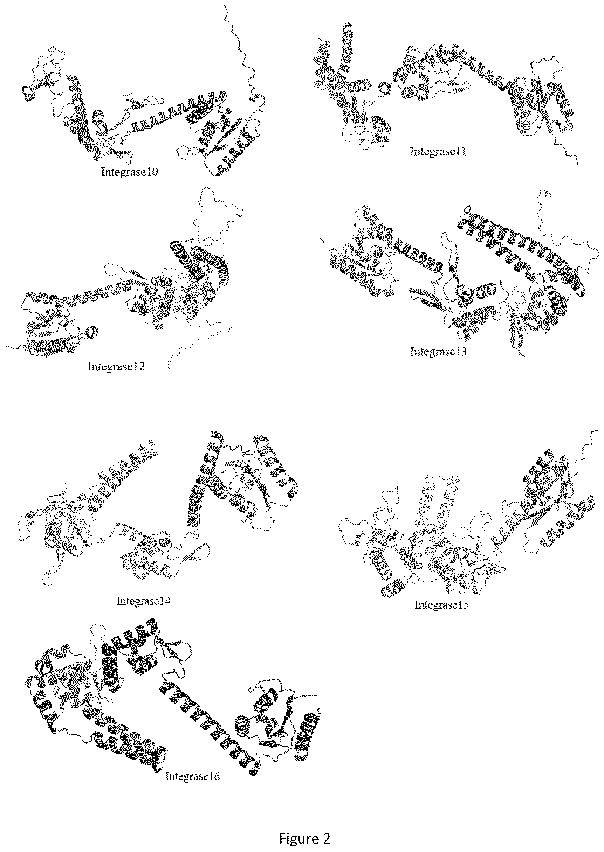

The structures of the above proteins were predicted using the AlphaFold server, and their three-dimensional structures were visualized using the molecular 3D structure visualization software PyMOL (https://pymol.org/2/). The three-dimensional structures of the integrases are shown in Figure 2.

### Example 2: Integration Identification Experiment of Integrases

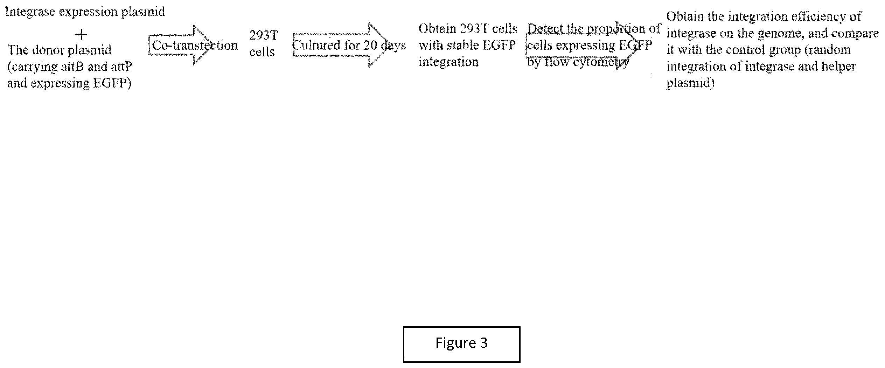

To verify whether the newly discovered integrases can mediate the integration of large-fragment DNA into mammalian genomes, the inventors designed and conducted verification experiments. The specific experimental design is shown in Figure 3.

The workflow of the identification experiment is as follows:

### 1. Construction of integrase expression plasmids

The 7 DNA sequences encoding integrases Cyin14, Cyin10, Cyin11, Cyin12, Cyin13, Cyin15, and Cyin16, were codon-optimized to obtain codon-optimized coding sequences SEQ ID No. 10-16, which are suitable for expression in human cells. The coding sequences of integrase phiC31 (amino acid sequence shown in SEQ ID No. 43) and integrase Bxb1 (amino acid sequence shown in SEQ ID No. 44) were also codon-optimized to obtain codon-optimized coding sequences suitable for expression in human cells, which are SEQ ID No. 45 and SEQ ID No. 46, respectively.

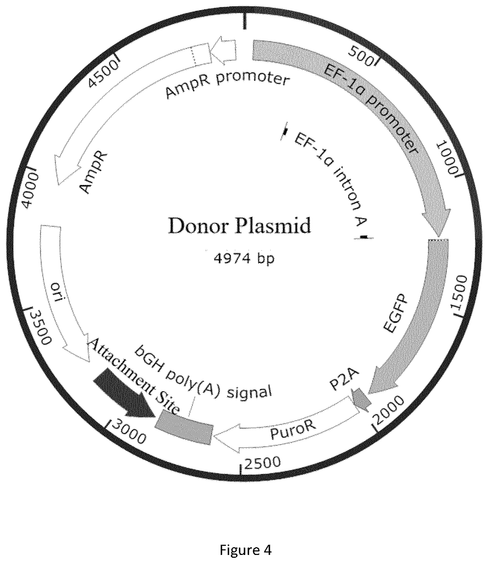

The DNA sequences SEQ ID No.10-16, 45, and 46 were artificially synthesized and double-digested using restriction endonucleases Notl (Thermo Fisher, Cat. No. FD0593) and Agel (Thermo Fisher, Cat. No. FD1464). A backbone vector ABE8e (purchased from Addgene, #138489) was digested with both of these restriction endonucleases to generate digested fragments of 3361 bp and 4856 bp, respectively. The 3361 bp backbone fragment was recovered by gel extraction using a PCR product recovery kit (purchased from Tiangen Biochemical Technology (Beijing) Co., Ltd.). Each double-digested DNA fragment was ligated to the backbone fragment recovered after double digestion of the ABE8e plasmid. After ligation, the region between base 409 and base 5226 of the ABE8e plasmid was replaced by sequence SEQ ID No. 10-16, 45, or 46, respectively, to obtain 9 integrase recombinant expression plasmids. The map of the integrase recombinant expression plasmid is shown in Figure 4, which was verified to be correct by Sanger sequencing.

### 2. Construction of donor plasmids expressing EGFP and carrying attachment sites

The integration sequences of integrases Cyin10, Cyin11, Cyin12, Cyin13, Cyin14, Cyin15, and Cyin16 were predicted using the Blast alignment tool on the NCBI website, and the obtained integration sequences of these integrases are shown in SEQ ID No. 17-42, respectively.

The EGFP donor plasmid (SEQ ID No. 8) and the integration sequences to be inserted into the donor plasmid (SEQ ID No.17-42, 47-48) were prepared by artificial synthesis. After synthesis, the sequence between base 2855 and base 2868 of the EGFP donor plasmid was replaced with each integration sequence by molecular cloning, thereby obtaining different donor plasmids for the integrases. The EGFP gene is regulated by the EF1α promoter.

### 3. Transformation with expression plasmids and integration site recombinant donor plasmids

*E. coli* DH5α competent cells (commercially available) were transformed with the constructed integrase expression plasmids and integration site recombinant donor plasmids. After plating, picking clones, and shaking cultures, plasmid extraction was performed.

### 4. Integration identification for integrases Cyin10-16

### (1) Design of groups for cell transfection experiment

The experimental groups were as follows: each integrase expression plasmid and its corresponding donor plasmid were co-transfected into 293T cells. The transfection reagent used was PEI (Polysciences, Cat. No. 23966-1). The corresponding integration sequences, integrase expression plasmids to be transfected, and corresponding donor plasmids containing integration sequences are shown in the following table.

**Table 2 Expression plasmids and corresponding donor plasmids to be transfected**

| Integrase Name | Corresponding Integration Sequence | Integration Sequence No. (SEQ ID No:) | Co-transfected Plasmids |
|---|---|---|---|
| Cyin10 | 10-P2 | 17 | Cyin10 expression plasmid + 10-P2 donor plasmid |
| Cyin10 | 10-B2 | 18 | Cyin10 expression plasmid + 10-B2 donor plasmid |
| Cyin11 | 11-P1 | 19 | Cyin11 expression plasmid + 11-P1 donor plasmid |
| Cyin11 | 11-B1 | 20 | Cyin11 expression plasmid + 11-B1 donor plasmid |
| Cyin11 | 11-P2 | 21 | Cyin11 expression plasmid + 11-P2 donor plasmid |
| Cyin11 | 11-B2 | 22 | Cyin11 expression plasmid + 11-B2 donor plasmid |
| Cyin12 | 12-P1 | 23 | Cyin12 expression plasmid + 12-P1 donor plasmid |
| Cyin12 | 12-B1 | 24 | Cyin12 expression plasmid + 12-B1 donor plasmid |
| Cyin12 | 12-P2 | 25 | Cyin12 expression plasmid + 12-P2 donor plasmid |
| Cyin12 | 12-B2 | 26 | Cyin12 expression plasmid + 12-B2 donor plasmid |
| Cyin13 | 13-P1 | 27 | Cyin13 expression plasmid + 13-P1 donor plasmid |
| Cyin13 | 13-B1 | 28 | Cyin13 expression plasmid + 13-B1 donor plasmid |
| Cyin13 | 13-P2 | 29 | Cyin13 expression plasmid + 13-P2 donor plasmid |
| Cyin13 | 13-B2 | 30 | Cyin13 expression plasmid + 13-B2 donor plasmid |
| Cyin14 | 14-P1 | 31 | Cyin14 expression plasmid + 14-P1 donor plasmid |
| Cyin14 | 14-B1 | 32 | Cyin14 expression plasmid + 14-B1 donor plasmid |
| Cyin14 | 14-P2 | 33 | Cyin14 expression plasmid + 14-P2 donor plasmid |
| Cyin14 | 14-B2 | 34 | Cyin14 expression plasmid + 14-B2 donor plasmid |
| Cyin15 | 15-P1 | 35 | Cyin15 expression plasmid + 15-P1 donor plasmid |
| Cyin15 | 15-B1 | 36 | Cyin15 expression plasmid + 15-B1 donor plasmid |
| Cyin15 | 15-P2 | 37 | Cyin15 expression plasmid + 15-P2 donor plasmid |
| Cyin15 | 15-B2 | 38 | Cyin15 expression plasmid + 15-B2 donor plasmid |
| Cyin16 | 16-P1 | 39 | Cyin16 expression plasmid + 16-P1 donor plasmid |
| Cyin16 | 16-B1 | 40 | Cyin16 expression plasmid + 16-B1 donor plasmid |
| Cyin16 | 16-P2 | 41 | Cyin16 expression plasmid + 16-P2 donor plasmid |
| Cyin16 | 16-B2 | 42 | Cyin16 expression plasmid + 16-B2 donor plasmid |
| phiC31 | P31-attB | 47 | phiC31 expression plasmid + attB |
| Bxb1 | B1-attP | 48 | Bxb1 expression plasmid + attP |

The control group was as follows: a control plasmid pUC19 (plasmid sequence was SEQ ID No. 49) that does not express an integrase and each donor plasmid were co-transfected into 293T cells.

### (2) Transfection experiment

293T cells were passaged into a 96-well plate at a density of 50,000 cells per well and cultured at 37°C, 5% CO₂ for 18 hours before transfection.

Transfection for the negative control group:
- 0.6 µl of PEI (purchased from Polysciences, Cat. No. 23966-1) at a concentration of 1 µg/µl was added to 5 µl of UltraFectin^{®} transfection-specific serum-reduced medium (OptiMEM) (purchased from Shanghai Yuanpei Biotechnology Co., Ltd., Cat. No. L530KJ), and mixed well to prepare Solution A.
- 50 ng of each donor plasmid containing an integration sequence and 100 ng of the control plasmid that does not express integrase (pUC19, plasmid sequence was SEQ ID No. 49) were added to 5 µl of UltraFectin^{®} transfection-specific serum-reduced medium, and mixed well to prepare Solution B.

After standing for 5 minutes, Solution A was added to Solution B, mixed gently, and left to stand for 30 minutes to form a transfection complex. The entire volume of the transfection complex was added to the corresponding wells of a 96-well plate containing cultured 293T cells. After 6 hours of transfection, the medium was replaced with a DMEM medium containing 10% fetal bovine serum and 1% double antibodies (15070063, Gibco). After 24 hours of transfection, the medium was replaced with a DMEM screening medium containing 10% fetal bovine serum and 10 µg/ml puromycin (Gibco, A1113803). After 48 hours, the medium was replaced back with a DMEM medium containing 10% fetal bovine serum, and culturing was continued at 37°C, 5% CO₂, with passaging every 2-3 days.

### Transfection for the positive control group:

0.6 µl of PEI (purchased from Polysciences, Cat. No. 23966-1) at a concentration of 1 µg/µl was added to 5 µl of UltraFectin^{®} transfection-specific serum-reduced medium (OptiMEM), and mixed well to prepare Solution A.

100 ng of the plasmid expressing integrase phiC31 and 50 ng of the donor plasmid containing the attB site (the integration sequence of phiC31) to 5 µl of Opti-MEM serum-reduced medium (Thermo, Cat. No. 31985088), and mixed well to prepare Solution B.

After standing for 5 minutes, Solution A was added to Solution B, mixed gently, and left to stand for 30 minutes to form a transfection complex. The entire volume of the transfection complex was added to 293T cells in the corresponding wells. After 6 hours of transfection, the medium was replaced with a DMEM medium containing 10% fetal bovine serum and 1% double antibodies (Gibco, Cat. No. 15070063). After 24 hours of transfection, the medium was replaced with DMEM screening medium containing 10% fetal bovine serum and 10 µg/ml puromycin (Gibco, Cat. No. A1113803). After 48 hours, the medium was replaced back with a DMEM medium containing 10% fetal bovine serum, and culturing was continued, with passaging every 2-3 days.

The same transfection procedure was applied to the plasmid expressing integrase Bxb1 and the donor plasmid containing the integration sequence of Bxb1. After 48 hours of transfection, the medium was also replaced back with a DMEM medium containing 10% fetal bovine serum, and culturing was continued at 37°C, 5% CO₂, with passaging every 2-3 days.

### Transfection for the experimental group:

0.6 µl of PEI (purchased from Polysciences, Cat. No. 23966-1) at a concentration of 1 µg/µl was added to 5 µl of UltraFectin^{®} transfection-specific serum-reduced medium (OptiMEM), and mixed well to prepare Solution A.

100 ng of each integrase plasmid and 50 ng of a corresponding donor plasmid were added to 5 µl of Opti-MEM serum-reduced medium (Thermo, Cat. No. 31985088), and mixed well to prepare Solution B.

After standing for 5 minutes, Solution A was added to Solution B, mixed gently, and left to stand for 30 minutes to form a transfection complex. The entire volume of the transfection complex was added to 293T cells in the corresponding wells. After 6 hours of transfection, the medium was replaced with a DMEM medium containing 10% fetal bovine serum and 1% double antibodies (Gibco, Cat. No. 15070063). After 24 hours of transfection, the medium was replaced with a DMEM screening medium containing 10% fetal bovine serum and 10 µg/ml puromycin (Gibco, Cat. No. A1113803). After 48 hours of transfection, the medium was replaced back with a DMEM medium containing 10% fetal bovine serum, and culturing was continued at 37°C, 5% CO₂, with passaging every 2-3 days.

### (3) Evaluation of integration efficiency

After 20 days of culturing, the medium in each well was discarded. The 293T cells in each well were digested with 80 µl of 0.05% trypsin by incubating at 37°C for 5 minutes, and then 80 µl of a medium containing 10% fetal bovine serum was added to terminate digestion. The cells were pipetted to form a single-cell suspension, and centrifuged at 400g for 5 minutes, and the supernatant was discarded. The cells were resuspended in 200 µl of a medium containing 10% FBS, and the proportion of EGFP-positive cells was measured by a flow cytometer. This proportion represents the integration efficiency of the mediated integration of large-fragment DNA into the genome.

After 20 days of cell culturing, the inventors measured the proportion of EGFP-positive cells by flow cytometry. The 293T cells successfully transfected with the integrase plasmids and donor plasmids were mainly divided into two categories. In one category, the donor plasmid was integrated into the genome, and these cells would continuously express EGFP protein. In the other category, the donor plasmid was not integrated into the genome, but was metabolized as the culturing time passed, thereby no longer expressing EGFP protein. Therefore, whether exogenous large-fragment DNA has been integrated can be characterized by detecting whether the 293T cells express EGFP protein, and the integration efficiency of large-fragment DNA can be obtained by analyzing the proportion of EGFP-positive cells.

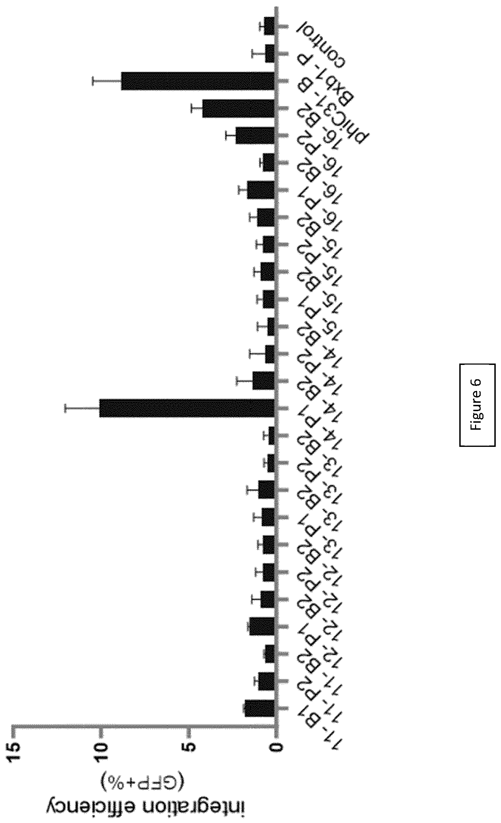

Figure 6 shows the efficiencies of integration of different donor plasmids into the genome of 293T cells mediated by the 7 integrases (integrase Cyin10 corresponded to two integration sequences; integrases Cyin11-16 each corresponded to three or four integration sequences). From Figure 6, the integration efficiency of donor plasmids into the genome when the integrases correspond to their attachment sequences can be seen, as shown in Table 3.

**Table 3 Integration Efficiency of Integrases and Corresponding Integration Sequences**

| Integrase Name | Integration Sequence | Integration Efficiency |
|---|---|---|
| Cyin10 | 10-P2 | 0.97% |
| Cyin10 | 10-B2 | 0.68% |
| Cyin11 | 11-B1 | 1.78% |
| Cyin11 | 11-P1 | 1.01% |
| Cyin11 | 11-B2 | 0.63% |
| Cyin12 | 12-P1 | 1.53% |
| Cyin12 | 12-B1 | 0.90% |
| Cyin12 | 12-P2 | 0.75% |
| Cyin12 | 12-B2 | 0.77% |
| Cyin13 | 13-P1 | 0.81% |
| Cyin13 | 13-B1 | 1.05% |
| Cyin13 | 13-P2 | 0.48% |
| Cyin13 | 13-B2 | 0.45% |
| Cyin14 | 14-P1 | 10.12% |
| Cyin14 | 14-B1 | 1.38% |
| Cyin14 | 14-P2 | 0.66% |
| Cyin14 | 14-B2 | 0.48% |
| Cyin15 | 15-P1 | 0.80% |
| Cyin15 | 15-B1 | 0.87% |
| Cyin15 | 15-P2 | 0.77% |
| Cyin15 | 15-B2 | 1.12% |
| Cyin16 | 16-P1 | 1.69% |
| Cyin16 | 16-B1 | 0.80% |
| Cyin16 | 16-P2 | 2.33% |
| Cyin16 | 16-B2 | 4.23% |
| phiC31 | P31-attB | 8.87% |
| Bxb1 | B1-attP | 0.26% |

As can be seen from Table 3, integrases Cyin10-16 exhibited integration efficiency when combined with specific integration sequences, especially when integrase Cyin14 is combined with integration sequence 14-P1, which can mediate the integration of approximately 5kb large fragment DNA into the mammalian genome with an integration efficiency as high as 10.12%, comparable to the integration level of integrase phiC31 which was reported to have high integration efficiency. Integrase Cyin11 combined with attachment sequence 11-B1, integrase Cyin12 combined with attachment sequence 12-P1, integrase Cyin14 combined with integration sequence 14-B1, integrase Cyin15 combined with attachment sequence 15-B2, and integrase Cyin16 combined with integration sequence 16-P1, 16-P2, or 16-B2, all showed relatively high integration efficiency. A comparison of integration efficiencies between the integrases and their corresponding integration sequences is shown in Figure 6.

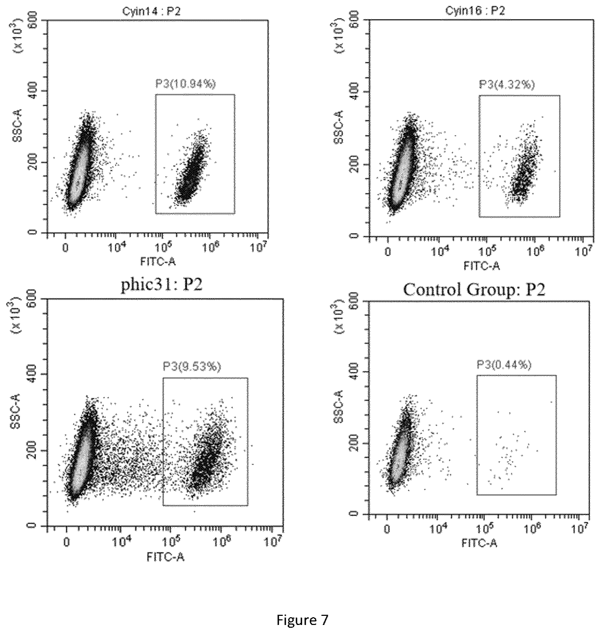

Figure 7 shows the flow cytometry analysis results of: integrase Cyin14 with the donor plasmid containing the 14-P1 integration sequence, integrase Cyin16 with the donor plasmid containing the 16-B2 integration sequence, the positive control group (integrase phiC31), and the negative control group (without an integrase expression plasmid, i.e., with random integration of the donor plasmid). It can be seen from the figure that, as compared with the control group, the integration efficiencies of integrases Cyin14, Cyin16, and phiC31 combined with their associated integration sequences are significantly higher than that of the control group.

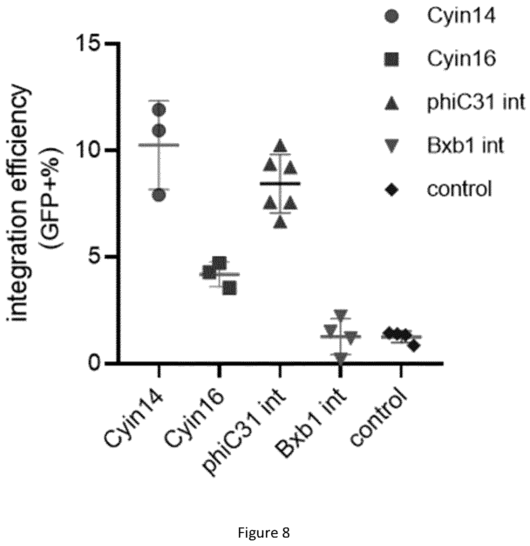

Figure 8 shows a comparison of integration efficiencies between integrases Cyin14, Cyin16 and the reported integrases phiC31 and Bxb1. From these results, it can be seen that integrases Cyin14 and Cyin16 can efficiently mediate effective integration of approximately 5 kb large fragment DNA into the mammalian genome.

All sequences used in the disclosure are described below and shown in Table 4.
SEQ ID No. 8-9 and 17-49 are as follows.
SEQ ID No. 8 (Donor plasmid sequence without integration sequence (EGFP donor plasmid)) 
GATCGGGAGATCGATCTCCCGATCCCCTAGGcacgtgggctccggtgcccgtcagtgggcagagcgcacatcgcccacagtc
cccgagaagttggggggaggggtcggcaattgaaccggtgcctagagaaggtggcgcggggtaaactgggaaagtgatgtcgtgtactgg
ctccgcctttttcccgagggtgggggagaaccgtatataagtgcagtagtcgccgtgaacgttctttttcgcaacgggtttgccgccagaaca
caggtaagtgccgtgtgtggttcccgcgggcctggcctctttacgggttatggcccttgcgtgccttgaattacttccacctggctgcagtacgt
gattcttgatcccgagcttcgggttggaagtgggtgggagagttcgaggccttgcgcttaaggagccccttcgcctcgtgcttgagttgaggcc
tggcctgggcgctggggccgccgcgtgcgaatctggtggcaccttcgcgcctgtctcgctgctttcgataagtctctagccatttaaaatttttg
atgacctgctgcgacgctttttttctggcaagatagtcttgtaaatgcgggccaagatctgcacactggtatttcggtttttgggccgcgggcg
gcgacggggcccgtgcgtcccagcgcacatgttcggcgaggcggggcctgcgagcgcggccaccgagaatcggacgggggtagtctcaag
ctggccggcctgctctggtgcctggcctcgcgccgccgtgtatcgccccgccctgggcggcaaggctggcccggtcggcaccagttgcgtgag
cggaaagatggccgcttcccggccctgctgcagggagctcaaaatggaggacgcggcgctcgggagagcgggcgggtgagtcacccacac
aaaggaaaagggcctttccgtcctcagccgtcgcttcatgtgactccactgagtaccgggcgccgtccaggcacctcgattagttctcgagct
tttggagtacgtcgtctttaggttggggggaggggttttatgcgatggagtttccccacactgagtgggtggagactgaagttaggccagcttg
gcacttgatgtaattctccttggaatttgccctttttgagtttggatcttggttcattctcaagcctcagacagtggttcaaagtttttttcttccat
ttcaggtgtcgtgatctagagATGGTGAGCAAGGGCGAGGAGCTGTTCACCGGGGTGGTGCCCATCCTGGTCGAG
CTGGACGGCGACGTAAACGGCCACAAGTTCAGCGTGTCCGGCGAGGGCGAGGGCGATGCCACCTACGGC
AAGCTGACCCTGAAGTTCATCTGCACCACCGGCAAGCTGCCCGTGCCCTGGCCCACCCTCGTGACCACCCT
GACCTACGGCGTGCAGTGCTTCAGCCGCTACCCCGACCACATGAAGCAGCACGACTTCTTCAAGTCCGCCA
TGCCCGAAGGCTACGTCCAGGAGCGCACCATCTTCTTCAAGGACGACGGCAACTACAAGACCCGCGCCGA
GGTGAAGTTCGAGGGCGACACCCTGGTGAACCGCATCGAGCTGAAGGGCATCGACTTCAAGGAGGACGG
CAACATCCTGGGGCACAAGCTGGAGTACAACTACAACAGCCACAACGTCTATATCATGGCCGACAAGCAG
AAGAACGGCATCAAGGTGAACTTCAAGATCCGCCACAACATCGAGGACGGCAGCGTGCAGCTCGCCGACC
ACTACCAGCAGAACACCCCCATCGGCGACGGCCCCGTGCTGCTGCCCGACAACCACTACCTGAGCACCCAG
TCCGCCCTGAGCAAAGACCCCAACGAGAAGCGCGATCACATGGTCCTGCTGGAGTTCGTGACCGCCGCCG
GGATCACTCTCGGCATGGACGAGCTGTACAAGgaattcggatccggcgcaacaaacttctctctgctgaaacaagccggaga
tgtcgaagagaatcctggaccgatgaccgagtacaagcccacggtgcgcctcgccacccgcgacgacgtccccagggccgtacgcaccctc
gccgccgcgttcgccgactaccccgccacgcgccacaccgtcgatccggaccgccacatcgagcgggtcaccgagctgcaagaactcttcct
cacgcgcgtcgggctcgacatcggcaaggtgtgggtcgcggacgacggcgccgccgtggcggtctggaccacgccggagagcgtcgaagc
gggggcggtgttcgccgagatcggcccgcgcatggccgagttgagcggttcccggctggccgcgcagcaacagatggaaggcctcctggcg
ccgcaccggcccaaggagcccgcgtggttcctggccaccgtcggagtctcgcccgaccaccagggcaagggtctgggcagcgccgtcgtgc
tccccggagtggaggcggccgagcgcgccggggtgcccgccttcctggagacctccgcgccccgcaacctccccttctacgagcggctcggc
ttcaccgtcaccgccgacgtcgaggtgcccgaaggaccgcgcacctggtgcatgacccgcaagcccggtgcctgaacgcgttaaCTGTGC
CTTCTAGTTGCCAGCCATCTGTTGTTTGCCCCTCCCCCGTGCCTTCCTTGACCCTGGAAGGTGCCACTCCCAC
TGTCCTTTCCTAATAAAATGAGGAAATTGCATCGCATTGTCTGAGTAGGTGTCATTCTATTCTGGGGGGTGG
GGTGGGGCAGGACAGCAAGGGGGAGGATTGGGAAGACAATAGCAGGCATGCTGGGGATGCGGTGGGCT
 
CTATGGctcaagcttGTGTGGCTGGTGggtaccACATGTGAGCAAAAGGCCAGCAAAAGGCCAGGAACCGTAA
AAAGGCCGCGTTGCTGGCGTTTTTCCATAGGCTCCGCCCCCCTGACGAGCATCACAAAAATCGACGCTCAA
GTCAGAGGTGGCGAAACCCGACAGGACTATAAAGATACCAGGCGTTTCCCCCTGGAAGCTCCCTCGTGCG
CTCTCCTGTTCCGACCCTGCCGCTTACCGGATACCTGTCCGCCTTTCTCCCTTCGGGAAGCGTGGCGCTTTCT
CATAGCTCACGCTGTAGGTATCTCAGTTCGGTGTAGGTCGTTCGCTCCAAGCTGGGCTGTGTGCACGAACC
CCCCGTTCAGCCCGACCGCTGCGCCTTATCCGGTAACTATCGTCTTGAGTCCAACCCGGTAAGACACGACTT
ATCGCCACTGGCAGCAGCCACTGGTAACAGGATTAGCAGAGCGAGGTATGTAGGCGGTGCTACAGAGTTC
TTGAAGTGGTGGCCTAACTACGGCTACACTAGAAGAACAGTATTTGGTATCTGCGCTCTGCTGAAGCCAGT
TACCTTCGGAAAAAGAGTTGGTAGCTCTTGATCCGGCAAACAAACCACCGCTGGTAGCGGTGGTTTTTTTG
TTTGCAAGCAGCAGATTACGCGCAGAAAAAAAGGATCTCAAGAAGATCCTTTGATCTTTTCTACGGGGTCT
GACACTCAGTGGAACGAAAACTCACGTTAAGGGATTTTGGTCATGAGATTATCAAAAAGGATCTTCACCTA
GATCCTTTTAAATTAAAAATGAAGTTTTAAATCAATCTAAAGTATATATGAGTAAACTTGGTCTGACAGTTA
CCAATGCTTAATCAGTGAGGCACCTATCTCAGCGATCTGTCTATTTCGTTCATCCATAGTTGCCTGACTCCC
GTCGTGTAGATAACTACGATACGGGAGGGCTTACCATCTGGCCCCAGTGCTGCAATGATACCGCGAGACCC
ACGCTCACCGGCTCCAGATTTATCAGCAATAAACCAGCCAGCCGGAAGGGCCGAGCGCAGAAGTGGTCCT
GCAACTTTATCCGCCTCCATCCAGTCTATTAATTGTTGCCGGGAAGCTAGAGTAAGTAGTTCGCCAGTTAAT
AGTTTGCGCAACGTTGTTGCCATTGCTACAGGCATCGTGGTGTCACGCTCGTCGTTTGGTATGGCTTCATTC
AGCTCCGGTTCCCAACGATCAAGGCGAGTTACATGATCCCCCATGTTGTGCAAAAAAGCGGTTAGCTCCTT
CGGTCCTCCGATCGTTGTCAGAAGTAAGTTGGCCGCAGTGTTATCACTCATGGTTATGGCAGCACTGCATA
ATTCTCTTACTGTCATGCCATCCGTAAGATGCTTTTCTGTGACTGGTGAGTACTCAACCAAGTCATTCTGAGA
ATAGTGTATGCGGCGACCGAGTTGCTCTTGCCCGGCGTCAATACGGGATAATACCGCGCCACATAGCAGA
ACTTTAAAAGTGCTCATCATTGGAAAACGTTCTTCGGGGCGAAAACTCTCAAGGATCTTACCGCTGTTGAG
ATCCAGTTCGATGTAACCCACTCGTGCACCCAACTGATCTTCAGCATCTTTTACTTTCACCAGCGTTTCTGGG
TGAGCAAAAACAGGAAGGCAAAATGCCGCAAAAAAGGGAATAAGGGCGACACGGAAATGTTGAATACTC
ATACTCTTCCTTTTTCAATATTATTGAAGCATTTATCAGGGTTATTGTCTCATGAGCGGATACATATTTGAAT
GTATTTAGAAAAATAAACAAATAGGGGTTCCGCGCACATTTCCCCGAAAAGTGCCACCTGACGTCGACG
SEQ ID No. 9 (Backbone vector sequence used for integrase expression plasmids) 
ATATGCCAAGTACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGGTGATGCGGTTTT
GGCAGTACATCAATGGGCGTGGATAGCGGTTTGACTCACGGGGATTTCCAAGTCTCCACCCCATTGACGTC
AATGGGAGTTTGTTTTGGCACCAAAATCAACGGGACTTTCCAAAATGTCGTAACAACTCCGCCCCATTGAC
GCAAATGGGCGGTAGGCGTGTACGGTGGGAGGTCTATATAAGCAGAGCTGGTTTAGTGAACCGTCAGATC
CGCTAGAGATCCGCGGCCGCTAATACGACTCACTATAGGGAGAGCCGCCACCATGAAACGGACAGCCGAC
 
GGAAGCGAGTTCGAGTCACCAAAGAAGAAGCGGAAAGTCTCTGAGGTGGAGTTTTCCCACGAGTACTGGA
TGAGACATGCCCTGACCCTGGCCAAGAGGGCACGGGATGAGAGGGAGGTGCCTGTGGGAGCCGTGCTGG
TGCTGAACAATAGAGTGATCGGCGAGGGCTGGAACAGAGCCATCGGCCTGCACGACCCAACAGCCCATGC
CGAAATTATGGCCCTGAGACAGGGCGGCCTGGTCATGCAGAACTACAGACTGATTGACGCCACCCTGTAC
GTGACATTCGAGCCTTGCGTGATGTGCGCCGGCGCCATGATCCACTCTAGGATCGGCCGCGTGGTGTTTGG
CGTGAGGAACTCAAAAAGAGGCGCCGCAGGCTCCCTGATGAACGTGCTGAACTACCCCGGCATGAATCAC
CGCGTCGAAATTACCGAGGGAATCCTGGCAGATGAATGTGCCGCCCTGCTGTGCGATTTCTATCGGATGCC
TAGACAGGTGTTCAATGCTCAGAAGAAGGCCCAGAGCTCCATCAACTCCGGAGGATCTAGCGGAGGCTCC
TCTGGCTCTGAGACACCTGGCACAAGCGAGAGCGCAACACCTGAAAGCAGCGGGGGCAGCAGCGGGGGG
TCAGACAAGAAGTACAGCATCGGCCTGGCCATCGGCACCAACTCTGTGGGCTGGGCCGTGATCACCGACG
AGTACAAGGTGCCCAGCAAGAAATTCAAGGTGCTGGGCAACACCGACCGGCACAGCATCAAGAAGAACCT
GATCGGAGCCCTGCTGTTCGACAGCGGCGAAACAGCCGAGGCCACCCGGCTGAAGAGAACCGCCAGAAG
AAGATACACCAGACGGAAGAACCGGATCTGCTATCTGCAAGAGATCTTCAGCAACGAGATGGCCAAGGTG
GACGACAGCTTCTTCCACAGACTGGAAGAGTCCTTCCTGGTGGAAGAGGATAAGAAGCACGAGCGGCACC
CCATCTTCGGCAACATCGTGGACGAGGTGGCCTACCACGAGAAGTACCCCACCATCTACCACCTGAGAAAG
AAACTGGTGGACAGCACCGACAAGGCCGACCTGCGGCTGATCTATCTGGCCCTGGCCCACATGATCAAGTT
CCGGGGCCACTTCCTGATCGAGGGCGACCTGAACCCCGACAACAGCGACGTGGACAAGCTGTTCATCCAG
CTGGTGCAGACCTACAACCAGCTGTTCGAGGAAAACCCCATCAACGCCAGCGGCGTGGACGCCAAGGCCA
TCCTGTCTGCCAGACTGAGCAAGAGCAGACGGCTGGAAAATCTGATCGCCCAGCTGCCCGGCGAGAAGAA
GAATGGCCTGTTCGGAAACCTGATTGCCCTGAGCCTGGGCCTGACCCCCAACTTCAAGAGCAACTTCGACC
TGGCCGAGGATGCCAAACTGCAGCTGAGCAAGGACACCTACGACGACGACCTGGACAACCTGCTGGCCCA
GATCGGCGACCAGTACGCCGACCTGTTTCTGGCCGCCAAGAACCTGTCCGACGCCATCCTGCTGAGCGACA
TCCTGAGAGTGAACACCGAGATCACCAAGGCCCCCCTGAGCGCCTCTATGATCAAGAGATACGACGAGCA
CCACCAGGACCTGACCCTGCTGAAAGCTCTCGTGCGGCAGCAGCTGCCTGAGAAGTACAAAGAGATTTTCT
TCGACCAGAGCAAGAACGGCTACGCCGGCTACATTGACGGCGGAGCCAGCCAGGAAGAGTTCTACAAGTT
CATCAAGCCCATCCTGGAAAAGATGGACGGCACCGAGGAACTGCTCGTGAAGCTGAACAGAGAGGACCT
GCTGCGGAAGCAGCGGACCTTCGACAACGGCAGCATCCCCCACCAGATCCACCTGGGAGAGCTGCACGCC
ATTCTGCGGCGGCAGGAAGATTTTTACCCATTCCTGAAGGACAACCGGGAAAAGATCGAGAAGATCCTGA
CCTTCCGCATCCCCTACTACGTGGGCCCTCTGGCCAGGGGAAACAGCAGATTCGCCTGGATGACCAGAAAG
AGCGAGGAAACCATCACCCCCTGGAACTTCGAGGAAGTGGTGGACAAGGGCGCTTCCGCCCAGAGCTTCA
TCGAGCGGATGACCAACTTCGATAAGAACCTGCCCAACGAGAAGGTGCTGCCCAAGCACAGCCTGCTGTA
CGAGTACTTCACCGTGTATAACGAGCTGACCAAAGTGAAATACGTGACCGAGGGAATGAGAAAGCCCGCC
TTCCTGAGCGGCGAGCAGAAAAAGGCCATCGTGGACCTGCTGTTCAAGACCAACCGGAAAGTGACCGTGA
AGCAGCTGAAAGAGGACTACTTCAAGAAAATCGAGTGCTTCGACTCCGTGGAAATCTCCGGCGTGGAAGA
 
TCGGTTCAACGCCTCCCTGGGCACATACCACGATCTGCTGAAAATTATCAAGGACAAGGACTTCCTGGACA
ATGAGGAAAACGAGGACATTCTGGAAGATATCGTGCTGACCCTGACACTGTTTGAGGACAGAGAGATGAT
CGAGGAACGGCTGAAAACCTATGCCCACCTGTTCGACGACAAAGTGATGAAGCAGCTGAAGCGGCGGAG
ATACACCGGCTGGGGCAGGCTGAGCCGGAAGCTGATCAACGGCATCCGGGACAAGCAGTCCGGCAAGAC
AATCCTGGATTTCCTGAAGTCCGACGGCTTCGCCAACAGAAACTTCATGCAGCTGATCCACGACGACAGCC
TGACCTTTAAAGAGGACATCCAGAAAGCCCAGGTGTCCGGCCAGGGCGATAGCCTGCACGAGCACATTGC
CAATCTGGCCGGCAGCCCCGCCATTAAGAAGGGCATCCTGCAGACAGTGAAGGTGGTGGACGAGCTCGTG
AAAGTGATGGGCCGGCACAAGCCCGAGAACATCGTGATCGAAATGGCCAGAGAGAACCAGACCACCCAG
AAGGGACAGAAGAACAGCCGCGAGAGAATGAAGCGGATCGAAGAGGGCATCAAAGAGCTGGGCAGCCA
GATCCTGAAAGAACACCCCGTGGAAAACACCCAGCTGCAGAACGAGAAGCTGTACCTGTACTACCTGCAG
AATGGGCGGGATATGTACGTGGACCAGGAACTGGACATCAACCGGCTGTCCGACTACGATGTGGACCATA
TCGTGCCTCAGAGCTTTCTGAAGGACGACTCCATCGACAACAAGGTGCTGACCAGAAGCGACAAGAACCG
GGGCAAGAGCGACAACGTGCCCTCCGAAGAGGTCGTGAAGAAGATGAAGAACTACTGGCGGCAGCTGCT
GAACGCCAAGCTGATTACCCAGAGAAAGTTCGACAATCTGACCAAGGCCGAGAGAGGCGGCCTGAGCGA
ACTGGATAAGGCCGGCTTCATCAAGAGACAGCTGGTGGAAACCCGGCAGATCACAAAGCACGTGGCACA
GATCCTGGACTCCCGGATGAACACTAAGTACGACGAGAATGACAAGCTGATCCGGGAAGTGAAAGTGATC
ACCCTGAAGTCCAAGCTGGTGTCCGATTTCCGGAAGGATTTCCAGTTTTACAAAGTGCGCGAGATCAACAA
CTACCACCACGCCCACGACGCCTACCTGAACGCCGTCGTGGGAACCGCCCTGATCAAAAAGTACCCTAAGC
TGGAAAGCGAGTTCGTGTACGGCGACTACAAGGTGTACGACGTGCGGAAGATGATCGCCAAGAGCGAGC
AGGAAATCGGCAAGGCTACCGCCAAGTACTTCTTCTACAGCAACATCATGAACTTTTTCAAGACCGAGATT
ACCCTGGCCAACGGCGAGATCCGGAAGCGGCCTCTGATCGAGACAAACGGCGAAACCGGGGAGATCGTG
TGGGATAAGGGCCGGGATTTTGCCACCGTGCGGAAAGTGCTGAGCATGCCCCAAGTGAATATCGTGAAAA
AGACCGAGGTGCAGACAGGCGGCTTCAGCAAAGAGTCTATCCTGCCCAAGAGGAACAGCGATAAGCTGAT
CGCCAGAAAGAAGGACTGGGACCCTAAGAAGTACGGCGGCTTCGACAGCCCCACCGTGGCCTATTCTGTG
CTGGTGGTGGCCAAAGTGGAAAAGGGCAAGTCCAAGAAACTGAAGAGTGTGAAAGAGCTGCTGGGGATC
ACCATCATGGAAAGAAGCAGCTTCGAGAAGAATCCCATCGACTTTCTGGAAGCCAAGGGCTACAAAGAAG
TGAAAAAGGACCTGATCATCAAGCTGCCTAAGTACTCCCTGTTCGAGCTGGAAAACGGCCGGAAGAGAAT
GCTGGCCTCTGCCGGCGAACTGCAGAAGGGAAACGAACTGGCCCTGCCCTCCAAATATGTGAACTTCCTGT
ACCTGGCCAGCCACTATGAGAAGCTGAAGGGCTCCCCCGAGGATAATGAGCAGAAACAGCTGTTTGTGGA
ACAGCACAAGCACTACCTGGACGAGATCATCGAGCAGATCAGCGAGTTCTCCAAGAGAGTGATCCTGGCC
GACGCTAATCTGGACAAAGTGCTGTCCGCCTACAACAAGCACCGGGATAAGCCCATCAGAGAGCAGGCCG
AGAATATCATCCACCTGTTTACCCTGACCAATCTGGGAGCCCCTGCCGCCTTCAAGTACTTTGACACCACCA
TCGACCGGAAGAGGTACACCAGCACCAAAGAGGTGCTGGACGCCACCCTGATCCACCAGAGCATCACCGG
CCTGTACGAGACACGGATCGACCTGTCTCAGCTGGGAGGTGACTCTGGCGGCTCAAAAAGAACCGCCGAC
 
GGCAGCGAATTCGAGCCAAGAAGAAGAGGAAAGTCTAACCGGTCATCATCACCATCACCATTGAGTTTA
AACCCGCTGATCAGCCTCGACTGTGCCTTCTAGTTGCCAGCCATCTGTTGTTTGCCCCTCCCCCGTGCCTTCC
TTGACCCTGGAAGGTGCCACTCCCACTGTCCTTTCCTAATAAAATGAGGAAATTGCATCGCATTGTCTGAGT
AGGTGTCATTCTATTCTGGGGGGTGGGGTGGGGCAGGACAGCAAGGGGGAGGATTGGGAAGACAATAG
CAGGCATGCTGGGGATGCGGTGGGCTCTATGGCTTCTGAGGCGGAAAGAACCAGCTGGGGCTCGATACC
GTCGACCTCTAGCTAGAGCTTGGCGTAATCATGGTCATAGCTGTTTCCTGTGTGAAATTGTTATCCGCTCAC
AATTCCACACAACATACGAGCCGGAAGCATAAAGTGTAAAGCCTAGGGTGCCTAATGAGTGAGCTAACTC
ACATTAATTGCGTTGCGCTCACTGCCCGCTTTCCAGTCGGGAAACCTGTCGTGCCAGCTGCATTAATGAATC
GGCCAACGCGCGGGGAGAGGCGGTTTGCGTATTGGGCGCTCTTCCGCTTCCTCGCTCACTGACTCGCTGCG
CTCGGTCGTTCGGCTGCGGCGAGCGGTATCAGCTCACTCAAAGGCGGTAATACGGTTATCCACAGAATCA
GGGGATAACGCAGGAAAGAACATGTGAGCAAAAGGCCAGCAAAAGGCCAGGAACCGTAAAAAGGCCGC
GTTGCTGGCGTTTTTCCATAGGCTCCGCCCCCCTGACGAGCATCACAAAAATCGACGCTCAAGTCAGAGGT
GGCGAAACCCGACAGGACTATAAAGATACCAGGCGTTTCCCCCTGGAAGCTCCCTCGTGCGCTCTCCTGTT
CCGACCCTGCCGCTTACCGGATACCTGTCCGCCTTTCTCCCTTCGGGAAGCGTGGCGCTTTCTCATAGCTCA
CGCTGTAGGTATCTCAGTTCGGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTAAGACACGACTTATCGCCACT
GGCAGCAGCCACTGGTAACAGGATTAGCAGAGCGAGGTATGTAGGCGGTGCTACAGAGTTCTTGAAGTG
GTGGCCTAACTACGGCTACACTAGAAGAACAGTATTTGGTATCTGCGCTCTGCTGAAGCCAGTTACCTTCG
GAAAAAGAGTTGGTAGCTCTTGATCCGGCAAACAAACCACCGCTGGTAGCGGTGGTTTTTTTGTTTGCAAG
CAGCAGATTACGCGCAGAAAAAAAGGATCTCAAGAAGATCCTTTGATCTTTTCTACGGGGTCTGACACTCA
GTGGAACGAAAACTCACGTTAAGGGATTTTGGTCATGAGATTATCAAAAAGGATCTTCACCTAGATCCTTTT
AAATTAAAAATGAAGTTTTAAATCAATCTAAAGTATATATGAGTAAACTTGGTCTGACAGTTACCAATGCTT
AATCAGTGAGGCACCTATCTCAGCGATCTGTCTATTTCGTTCATCCATAGTTGCCTGACTCCCCGTCGTGTA
GATAACTACGATACGGGAGGGCTTACCATCTGGCCCCAGTGCTGCAATGATACCGCGAGACCCACGCTCAC
CGGCTCCAGATTTATCAGCAATAAACCAGCCAGCCGGAAGGGCCGAGCGCAGAAGTGGTCCTGCAACTTT
ATCCGCCTCCATCCAGTCTATTAATTGTTGCCGGGAAGCTAGAGTAAGTAGTTCGCCAGTTAATAGTTTGCG
CAACGTTGTTGCCATTGCTACAGGCATCGTGGTGTCACGCTCGTCGTTTGGTATGGCTTCATTCAGCTCCGG
TTCCCAACGATCAAGGCGAGTTACATGATCCCCCATGTTGTGCAAAAAAGCGGTTAGCTCCTTCGGTCCTCC
GATCGTTGTCAGAAGTAAGTTGGCCGCAGTGTTATCACTCATGGTTATGGCAGCACTGCATAATTCTCTTAC
TGTCATGCCATCCGTAAGATGCTTTTCTGTGACTGGTGAGTACTCAACCAAGTCATTCTGAGAATAGTGTAT
GCGGCGACCGAGTTGCTCTTGCCCGGCGTCAATACGGGATAATACCGCGCCACATAGCAGAACTTTAAAA
GTGCTCATCATTGGAAAACGTTCTTCGGGGCGAAAACTCTCAAGGATCTTACCGCTGTTGAGATCCAGTTC
GATGTAACCCACTCGTGCACCCAACTGATCTTCAGCATCTTTTACTTTCACCAGCGTTTCTGGGTGAGCAAA
AACAGGAAGGCAAAATGCCGCAAAAAAGGGAATAAGGGCGACACGGAAATGTTGAATACTCATACTCTTC
 
CTTTTTCAATATTATTGAAGCATTTATCAGGGTTATTGTCTCATGAGCGGATACATATTTGAATGTATTTAGA
AAAATAAACAAATAGGGGTTCCGCGCACATTTCCCCGAAAAGTGCCACCTGACGTCGACGGATCGGGAGA
TCGATCTCCCGATCCCCTAGGGTCGACTCTCAGTACAATCTGCTCTGATGCCGCATAGTTAAGCCAGTATCT
GCTCCCTGCTTGTGTGTTGGAGGTCGCTGAGTAGTGCGCGAGCAAAATTTAAGCTACAACAAGGCAAGGC
TTGACCGACAATTGCATGAAGAATCTGCTTAGGGTTAGGCGTTTTGCGCTGCTTCGCGATGTACGGGCCAG
ATATACGCGTTGACATTGATTATTGACTAGTTATTAATAGTAATCAATTACGGGGTCATTAGTTCATAGCCC
ATATATTGAGTTCCGCGTTACATAACTTACGGTAAATGGCCCGCCTGGCTGACCGCCCAACGACCCCCGCCC
ATTGACGTCAATAATGACGTATGTTCCCATAGTAACGCCAATAGGGACTTTCCATTGACGTCAATGGGTGG
AGTATTTACGGTAAACTGCCCACTTGGCAGTACATCAAGTGTATC
SEQ ID No.17(10-P2) 
GTTTTGAATGGACGAGTAAATCTATTCTTGCTGAGAAAAAATGCAACCACAATAGTTTTGACGGTACAAATT
ATACTGTTTTGATAAATCTACAGAAGATTTATAGCCATTATTTTCTTAAAATCATTTTCCAATAAAATAATCC
TTATTTTCAATCATTGCATTAAAAGTTTCAACAAAAGCTTGTTATAAAACTCTATCATCTATATGCTTGTTTTC
ACAACTCTTTTTCCCCTTTACTTCATACTTCC
SEQ ID No.18(10-B2) 
TCATCTTCTCTACTATCTTAAAAAACAAATCCATATCAAATTCTTCCAAAATTTCTGCATTTTCAATAACCTTT
ATAAACTGCTTAGCCTTATACTTCTTAAGAACATCTTCACTCTCTCCCTTCCAGAATTAAGAAATTTAATATTA
TATTTGTTCTCTAATTCTAATCCAATTTCATTTATTTTGGCTGCATTTTTCAAAGGACTTATTGTTAGTGTCGT
AGTAAAATAGTCAAATCCATTTGTTTTACC
SEQ ID No.19(11-P1) 
TTGTAATCCATGTCGAGGCCTTCCTCGGTGGATTTTCGGGTGTAGATTGCACAGCGTAGGATTTTCTTCTCC
TCCATCATGCCCGTGCCTTTCTGGCGCGATGATAGGGTCGCTTTTCCTTATCCTCGGATAAAATTTTTCGAT
CTATCATATTCCAGCGATCACATTATCTCCCAAAAATCCTGTTTCTTATTGGTTTCCCTATCTTGGACATGCCA
CTCTGCGCATTGCTATTTCAGATACAAAAATCT
SEQ ID No.20(11-B1) 
ATCCAATGTGCAAGGCCATTTAGATGTCGCTTTGGCAAACACACTCGGCCGTTTGTGTCATGAATTATAATA
TCAGACATGCTACTGAAAAAGATTTATCTGCACTTTGCGATATTGAACATGAATCTTTATTTCCCAAACCAAA
AAATGCGTTACCGTTCCAGCGCGTGCCGGTAATTGCAAATGCGACACCAGATAGGCTTTTATATTTCAGCCC
CTGATATTCATAGCCGTCAGCCAGCACATACACAT
SEQ ID No.21(11-P2) 
ATGGCTTCGACATATTTTCACCAGCCTCGCGCTGGGCATCCAGCGTGTTGTAATCCATGTCGAGGCCTTCC
TCGGTGGATTTTCGGGTGTAGATTGCACAGCGTAGGATTTCTTCTCCTCCATCATTAGGACATTACGATAT
TCATACGCCTGCAACGCGGTATGTCCTGTCACTTAATCACTATGTCCCAAACCCAGGGTCTTCTGATTCCAG
TCTGGTTGAGTCACTTCAACAGAATGGGTTGGACATT
SEQ ID No.22(11-B2) 
TATTGGCCAGACTCTGTATGCCGATTCTGCTGGTTATCACAGGCTCAAACAAGATTCTCATTTGCAAACATT
TATGGCGCATCACATCCAAAAGCACCATAATACGGTATCTGATAAGCACAGACCATGCCCGTGCCTTTCTG
GCGCGATGATAGGGTCGCTTTTCCTTATCCTCTTTATTTCCCAAACCAAAAAATGCGTTACCGTTCCAGCGC
GTGCCGGTAATTGCAAATGCGACACCAGATAGGCTTTT
SEQ ID No.23(12-P1) 
TTGTCTTCCTCTGCAGTCAATTGTTTTGGATCATCTTTTTTGGGAACAACAAGATTACTTTGAACATTATCTCT
ATTTACACCGGTTGAAACCTGGTCCTTCTTATTAAGAACTTCTGTATTTGTCAAAGCAATTCTTTATGTCAGA
GTGAGTACAGACGAACAGGCTGAAAAAGGTCACAGTCTGGCTCATCAGGAAGAACGACTTCGTAATTATT
GCAAAACAAATAATATCGAGGTCGTTGCTTTTTTTA
SEQ ID No.24(12-B1) 
ATGAAGAATTATTAAAAGTGAGAGAGTGGTTATATGTAGTCACTTCAATAATCATTAAGGTAATAAAGGAA
CAAGGAAAACAAACACCAGTTATAGACTTACAATCTGAAAATTATGAAAACAAGCAAGTTGTTTACATCTTT
TTTAGTTTCATTGTTTGTTCTGATCTCATTATTTAACTCGTTCTTTCCATTATCAACAATAAGGTGAACTTCTTT
TTCAATCAGTGATTCGTCATTTATTTTCTTTTGTC
SEQ ID No.25(12-P2) 
TGCCCATTGACCATGATTCCATGGACCCAATACAATATAATTGCGATTGAAACTGTCTTTTTCTCCATGTGG
TGATACATCAATTGCGGGCCATTAATATCTTCCTGGTCATAATAGCCGCCTACATTAAGGTAATAAAGGAAC
AAGGAAAACAAACACCAGTTATAGACTTACAATCTGAAAATTATGAAAACAAGCAAAGCAATTCTTTATGT
CAGAGTGAGTACAGACGAACAGGCTGAAAAAGGTCAC
SEQ ID No.26(12-B2) 
GATGTTTTTCCCGAAAGAATGAGTTTGGAAGAATTAAAAGAAATCTGGAATGATGAACATGGTTCTTTAAC
AGATGAAGAATTATTAAAAGTGAGAGAGTGGTTATATGTAGTCACTTCAATAATCATGTAATTGAGGTATT
 
TGCGGATAACTAACATAACTCAGCGGAGAATTTTTTTGCCAGAACTCGTCATAGTTAGGATGTGCCACAAA
ATTGTTCCAGGTTGGAATTTTATTATGAAAATATTTTTCA
SEQ ID No.27(13-P1) 
TTCGATATATGCCGAAAAATTAGTTTTGAAAATAATTCGTATCGAACTCCCGTATATAAAGAAGAGCTTTC
CCTAATACTCAATACTGGTAGGAGTTTTGGCGGAAATAAAAAAGGACAAAATCAAAAGACTGTATTCTTGC
GGCTTCCCGAACAGATATAGAACGCACTTGTTTTAAGTCAGGATATATGTAGTAATGACCGTCTTTGGAGA
TATGGGCGACTACGGTATGAGAATGACCAAGAGGATCGAC
SEQ ID No.28(13-B1) 
GTGTAGCCATTAAGTAACAGATGTTTTAATGACGCTGCGACTTTTTCAGCCATTAATGGCGGCACTGCGTTT
CCAATTTGTTTAAAAGCTGCGGTTCTACCACCCTCAAAGAAAAAATCATCTGGAAAAAAATTTGATTTTGTC
CTCTGGGGTAGCCCCGACAGGAATCGAACCTGTATCTAAAGTTTAGGAAACTTCTATTCTATCCATTGAACT
ACGGGGCCGATGATGAAACAGCAAAAATAATAGTTTCC
SEQ ID No.29(13-P2) 
AAAAATTAGTTTTTGAAAATAATTCGTATCGAACTCCCGTATATAAAGAAGAGCTTTCCCTAATACTCAATA
CTGGTAGGAGTTTTGGCGGAAATAAAAAAGGACAAAATCAAAAAAATTTGATTTTAAGTAAGTTGTCATTA
GTATATCCTGTTCCCGTTATGGCAGTTCGCTTTCCTGAGTTTTCTTGCAAGGTTACTCCCACTAATGTTTGAA
ATTTTCCTTTATTTCCAACTTTACAACTGTAATTGA
SEQ ID No.30(13-B2) 
TTCTACCTGTACAATTGACCAGGTATTTTTCGTCCCAGTTATAGTTAACCCTTCCAAATACGGCAGCATAATG
ATATAAATCATTTACATTATTAGTGAGTACTGTTTGAGCGCCCAAAGTTGAATTGTCCTCTGGGGTAGCCCC
GACAGGAATCGAACCTGTATCTAAAGTTTAGGAAACTTCTATTCTATCCATTGAACTACGGGGCCGATGAT
GAAACAGCAAAAATAATAGTTTCCCTTCACTGGGTA
SEQ ID No.31(14-P1) 
GGGTATTCGGCCTCTCTCCCCGTCACGTCCGCGACGTCTTCGTCGCCGGGGAGCTGGTGGTGGAAGACCG
GCGGCCAACGCGGGTCCAGCCGGAGGACATCGTCTGGGACGCCGCCGCGGAGGCCGCGCCGACCGCGGC
TGGGAGCTCGCCGAGACCTTCGAGGACGTGGACCGCTCCGGGTGGAACGGGGTTCACCGGCCCGGGCTC
GACCGGATGCTGGCCTCACTCGACGGCGTTGGCGCGCTGGTCGT
SEQ ID No.32(14-B1) 
ACGTATACTGGCGTACACTGATCAAGGAGGGGCCATGGCCAAGGCAGCCGGGTACGTCAGGCTCAGCAA
GTACGACGACGCCACCACTAGTCTCGCCCGCCAGCGTGAGGCCATCGAAAAGCTGTGCCCGGCTCTGGGA
TCGCCTCACCGAGATCGAGCCGCACGCGTTCGAACCGGTGGAGACGAGGTGAAGCCGAACAGGGTCGCG
CTCTATCTCCAAGACAAGCACCCCCTCCGCGAGGGGATGAAGTA
SEQ ID No.33(14-P2) 
GCGGGGGGCTTCATCTGCGGGCGGTTGGCGAAAACGCCGCCACCGCCGACGCGATGGGGGTGTCGGTCA
CCGCCTTTCGCTACGGGGCGGTGGTGCTCGGTGGAGCGCTCGCCGGACTGGCCGGCGCGCCGACCGCGG
CTGGGAGCTCGCCGAGACCTTCGAGGACGTGGACCGCTCCGGGTGGAACGGGGTTCACCGGCCCGGGCT
CGACCGGATGCTGGCCTCACTCGACGGCGTTGGCGCGCTGGTCGT
SEQ ID No.34(14-B2) 
ACGTATACTGGCGTACACTGATCAAGGAGGGGCCATGGCCAAGGCAGCCGGGTACGTCAGGCTCAGCAA
GTACGACGACGCCACCACTAGTCTCGCCCGCCAGCGTGAGGCCATCGAAAAGCTGTGCCTACCTTTCCCTT
GCGCTCACGCCGGGATGGAACGAGGGGATGACTGCCGGCCGAGGATGGATCGCTATCGGCCTTGTCATCT
TCGCCCGGTGGAATCCCATCCTGGCATTGGTCGGCGGGTACT
SEQ ID No.35(15-P1) 
TGAACTCCGGCACGCCAGCAGGGACCTTCTCACCTTGTGCGGGCGTGGTGACCAGTCGAACCCTGTCGCCG
GTCTGCAGCACCAGTCCCGGTGCTTGCGCGGCGGTGAGCGCCACGCCGACAACGGTCCTTGTCAGCGGCG
CCACCCAGGCCGGCAACTCCACTTGGCGCATGTGGCGCACCTGGTTGGTGGAGGTGGCTCTCGATGAATA
CGGCAACAGCAGTGCAAGAGCAGGTTCTGGTGTACTTGCGG
SEQ ID No.36(15-B1) 
TCTCGGCCGTCAACATCCGCAAGTTCGTGGTGCGAGCATCGCGCCTGTCGGAGCTGGTCGATCTGCAGAGC
CTCACGCCGTCCGCGGCCCTGTTCCTCGAGGCATCCATCCGGGCTGGGCTCAACGTGTTGCCGTCAGCCGG
GACGACGGCCCCGGAGAGCGATCCCGCTGTGAGCAGGCCGCCCTCGGCAATGTCGAGCGCCGCATGCTG
GCCGACCACCTGTTCGATCTCGCTTGCGAGCACCGGCTTCA
SEQ ID No.37(15-P2) 
GCCAGCAGGGACCTTCTCACCTTGTGCGGGCGTGGTGACCAGTCGAACCCTGTCGCCGGTCTGCAGCACCA
GTCCCGGTGCTTGCGCGGCGGTGAGCGCCACGCCGACAACGGTGTTGCCGTCAGCCGGGCTGGGCTCAAC
GTCCTTGTCAGCGGCGCCACCCAGGCCGGCAACTCCACTTGGCGCATGTGGCGCACCTGGTTGGTGGAGG
TGGCTCTCGATGAATACGGCAACAGCAGTGCAAGAGCAGGTTCT
SEQ ID No.38(15-B2) 
GGAATCAGCCGGGGCTTCTCGGCCGTCAACATCCGCAAGTTCGTGGTGCGAGCATCGCGCCTGTCGGAGC
TGGTCGATCTGCAGAGCCTCACGCCGTCCGCGGCCCTGTTCCTCGAGGCATCCATCCGGGACGACGGCCCC
GGAGAGCGATCCCGCTGTGAGCAGGCCGCCCTCGGCAATGTCGAGCGCCGCATGCTGGCCGACCACCTGT
TCGATCTCGCTTGCGAGCACCGGCTTCACCGCTGGGTCCGCGCT
SEQ ID No.39(16-P1) 
TCGATTCGGTTCGCCGCGCCGCTACCGGATCGCGCCGAAGAGATGACTGATCTCGAGGCTCCGGCCCGAA
GGCCTCCTCAACGGCTTCGAGGATCGATTCGGCGCGCACGGGTGCCTCTCTCCGCGCGCCAGCGCGAGGC
AATCGAGCGCCTCTGTGCTGACCGCGGGTGGGAGCTCGTTCGAGTCTTCGAGGATCTCGACCTCTCGGCCT
ACCGCCGCGACGTGAAGCGGCCGGGCTTCGAGGCCATGATGG
SEQ ID No.40(16-B1) 
GGCCGATTATTGACTGAACCGTAGACCAGCCCTATGCTGCTATGGCCGACCAGAGGAGGGGGAGCCATGC
GCGCCTTCGGCTACGTCCGGATCTCCAAGCTCGACGCCGACACGACCAGCCCACAGCGTCGCTGGTGGGC
GTCTCGGTCACGACACCGCTTTCCCTCGAATGACAACGGTGGCTGATTCCAGCACTAAATCAAGCATTACAC
GGCGCCGACAACCGCCGAAAGGCTCGAGGATAGCTACTTCC
SEQ ID No.41(16-P2) 
AGCGCCACTTCGTGTGGGGCGTGTTGGAGGATCTCCAAAGCCTCTTCGGCTGAGCCCGCCTCCGTGACGTC
GGCGCCCTCCACGCAGTTCTGCAGGACCTGGATGAGGCCCTTTCGAACCAGCGTGTGTACGTGGAGCGGA
AGGGGCCGATTATTGACTGAACCGTAGACCAGCCCTATGCTGCTATGGCCGACCAGAGGAGGGGGAGCCA
TGCGCGCCTTCGGCTACGTCCGGATCTCCAAGCTCGACGCCG
SEQ ID No.42(16-B2) 
TGCCGGCCAGTGCCGTCTCGGAGGTGGTGAACCAGGTTTCGGATGAAGTCGTCGGCCTCGGTCCCATCGA
GCGATTGCTCAAGGACCCGGAGATCAGCGAGGTGATGGTGAACGGGGCCGACGACGTGGTCGTCGACCA
GGAGGACCTTCACGAGGCCGCCTCTCGCAACGGGAACCGGGCCACGACCCGCGTGCCCTTCCCCGGCCGC
GGATAGACGTAGAGCCTGCCCCCAGCCTCCCGCACGCGGCGGCG
SEQ ID No. 43 Amino acid sequence of integrase phiC31 
MDTYAGAYDRQSRERENSSAASPATQRSANEDKAADLQREVERDGGRFRFVGHFSEAPGTSAFGTAERPEFER
ILNECRAGRLNMIIVYDVSRFSRLKVMDAIPIVSELLALGVTIVSTQEGVFRQGNVMDLIHLIMRLDASHKESSLKS
 
AKILDTKNLQRELGGYVGGKAPYGFELVSETKEITRNGRMVNVVINKLAHSTTPLTGPFEFEPDVIRWWWREIK
THKHLPFKPGSQAAIHPGSITGLCKRMDADAVPTRGETIGKKTASSAWDPATVMRILRDPRIAGFAAEVIYKKKP
DGTPTTKIEGYRIQRDPITLRPVELDCGPIIEPAEWYELQAWLDGRGRGKGLSRGQAILSAMDKLYCECGAVMT
SKRGEESIKDSYRCRRRKVVDPSAPGQHEGTCNVSMAALDKFVAERIFNKIRHAEGDEETLALLWEAARRFGKL
TEAPEKSGERANLVAERADALNALEELYEDRAAGAYDGPVGRKHFRKQQAALTLRQQGAEERLAELEAAEAPKL
PLDQWFPEDADADPTGPKSWWGRASVDDKRVFVGLFVDKIVVTKSTTGRGQGTPIEKRASITWAKPPTDDDE
DDAQDGTEDVAA*
SEQ ID No. 44 Amino acid sequence of integrase Bxb1 






*
SEQ ID No. 45 Nucleotide sequence of integrase phiC31 
ATGGATACCTACGCAGGCGCCTACGACAGACAGAGCAGAGAGAGAGAACAGCAGCGCCGCTTCTCCA
GCCACACAGAGATCCGCCAACGAGGACAAGGCAGCCGACCTGCAGAGAAGTGGAGAGACGGCGG
CAGGTTCAGATTCGTGGCCACTTTAGCGAAGCCCCAGGAACAAGCGCCTTTGGAACCGCCGAAAGACCC
GAGTTCGAGAGGATCCTCAACGAGTGTAGAGCCGGCCGGCTGAACATGATCATCGTGTACGACGTGTCCC
GGTTCAGCCGGCTGAAAGTGATGGACGCCATCCCAATCGTGTCCGAACTGCTGGCTCTGGGAGTGACCAT
CGTGTCTACCCAGGAGGGAGTGTTCAGGCAGGGCAACGTGATGGACCTGATCCACCTGATCATGCGGCTG
GACGCTTCTCACAAGGAGAGCAGCCTGAAGAGCGCCAAGATCCTGGACACCAAGAACCTGCAGCGGGAA
CTGGGAGGATACGTGGGAGGAAAGGCCCCTTACGGATTCGAGCTGGTGTCCGAGACCAAGGAGATCACC
AGGAACGGCCGGATGGTGAACGTCGTGATCAACAAGCTGGCCCACAGCACAACCCCTCTGACCGGCCCTT
TCGAGTTCGAACCAGACGTGATCAGGTGGTGGTGGAGGGAGATCAAGACCCACAAGCACCTGCCCTTCAA
GCCAGGATCTCAGGCCGCCATTCACCCAGGAAGCATCACCGGCCTCTGCAAGAGGATGGACGCTGACGCC
GTGCCTACAAGAGGAGAAACCATCGGCAAGAAGACAGCCTCTAGCGCCTGGGATCCAGCTACCGTGATGC
GGATCCTGAGGGATCCTAGAATCGCCGGATTTGCCGCCGAGGTCATCTACAAGAAGAAGCCCGACGGCAC
ACCTACCACCAAGATCGAGGGCTACCGGATCCAGAGGGACCCTATCACCCTGAGACCCGTGGAGCTGGAT
TGCGGACCCATCATCGAGCCAGCCGAGTGGTACGAACTGCAGGCTTGGCTGGATGGCAGAGGAAGAGGC
AAGGGACTGAGCAGAGGACAGGCTATCCTGAGCGCCATGGACAAGCTGTATTGCGAGTGCGGGGCAGTG
 
ATGACCAGCAAGAGGGGCGAGGAGAGCATCAAGGACAGCTACCGCTGCAGGAGGAGGAAGGTGGTGGA
TCCTTCAGCTCCAGGACAGCACGAGGGAACTTGCAACGTGTCCATGGCCGCTCTGGACAAGTTCGTGGCCG
AGCGCATCTTCAACAAGATCCGGCACGCCGAAGGAGACGAGGAAACACTGGCTCTCCTCTGGGAGGCAGC
TAGGAGATTCGGCAAGCTGACCGAGGCTCCAGAGAAAGCGGCGAGAGAGCCAACCTGGTGGCAGAAAG
AGCCGACGCTCTGAACGCTCTGGAGGAGCTGTACGAGGATAGAGCCGCAGGAGCTTACGACGGACCAGT
GGGCAGAAAGCACTTCAGGAAGCAGCAGGCCGCTCTGACACTGAGACAGCAGGGAGCCGAAGAGAGACT
GGCAGAACTGGAAGCCGCCGAAGCTCCTAAGCTGCCTCTCGATCAGTGGTTCCCCGAAGACGCAGACGCC
GATCCTACAGGCCCTAAGTCTTGGTGGGGCAGAGCTAGCGTGGACGATAAGAGGGTCTTCGTCGGCCTGT
TCGTGGACAAGATCGTGGTGACCAAGAGCACCACAGGCAGAGGACAGGGAACCCCTATCGAGAAGAGAG
CCAGCATCACCTGGGCCAAGCCTCCTACAGACGACGACGAAGACGACGCTCAGGACGGAACAGAGGACG
TGGCAGCTTAG
SEQ ID No. 46 Nucleotide sequence of Bxb1 
atgcgggccctggttgtgattagactgtccagagtgaccgacgccaccacctcacctgagagacagttggagagctgccagcagctgtgcgc
ccagcgcggctgggacgtggtgggcgtggccgaggacctggacgtgtccggcgccgtggacccttttgaccggaagcggagacctaatctg
gccagatggctggccttcgaggaacagcctttcgatgtgatcgtggcctacagagtggaccggctgaccagaagcatccggcacctgcaac
agctggtgcactgggccgaggatcacaaaaagctggtggtctctgctacagaggcccacttcgacacaaccacacctttcgccgctgtggtg
atcgccctgatggggaccgtggcccagatggaactcgaggccatcaaggaacggaacagatctgccgcccactttaacatcagagccggca
agtacagaggaagcctgccaccttggggctacctgcctaccagagttgacggcgagtggagactggtccccgaccccgtgcagagagagc
ggatcctggaggtgtaccacagagtggtcgacaaccacgagcccctgcatctggtggctcatgatctgaatagaaggggcgtgctgagccct
aaggactacttcgctcagctgcagggcagagaaccccagggcagagagtggagcgccaccgccctgaagagaagcatgatcagcgaggc
catgctgggatacgccaccctgaacggcaaaaccgtgcgggacgatgatggagcccctctggtgcgggcagagcctatcctaacccgcgag
cagctggaagctctgagagccgagctggtcaagacatctagagccaaacctgctgtgtccacccctagcctgctgctgcgggtgctgttctgt
gccgtgtgcggtgaacctgcttataagttcgccggcggcggcagaaagcaccccagataccggtgcagatctatgggcttccccaagcactg
tggcaacggcaccgtggccatggccgagtgggatgcttttgcgaggaacaagtgctggacctgctgggcgatgccgaaagactagagaag
gtgtgggtggctggcagcgacagcgccgtggaactggctgaagtgaacgccgagctggtggacctcaccagcctcatcggaagcccggcct
accgggccggctctccacagagagaagccctggatgcccgtatcgccgccctggccgctaggcaggaggaactggaaggcctggaagcca
gaccaagcggctgggagtggagagagacaggacagcggttcggagattggtggcgggaacaggatacagccgcaaagaacacctggctg
agaagcatgaacgtgcggctgacattcgacgtgagaggcggactgaccagaacaatcgacttcggcgacctgcaggagtacgagcaacac
ctgagactgggcagcgtggtcgagcggctgcacacaggcatgagctag
SEQ ID No. 47 Integration sequence attB of integrase phiC31 
GTCGACGATGTAGGTCACGGTCTCGAAGCCGCGGTGCGGGTGCCAGGGCGTGCCCTTGGGCTCCCCGGGC
GCGTACTCCACCTCACCCATCTGGTCCATCATGATGAACGGGTCGAGGTGGCGGTAGTTGATCCCGGCGAA
 
CGCGCGGCGCACCGGGAAGCCCTCGCCCTCGAAACCGCTGGGCGCGGTGGTCACGGTGAGCACGGGACG
TGCGACGGCGTCGGCGGGTGCGGATACGCGGGGCAGCGTCAGCGGGTTCTCGACGGTCACGGCGGGCAT
GTCGAC
SEQ ID No. 48 Integration sequence attP of integrase Bxb1
   CGTGGTTTGTCTGGTCAACCACCGCGGACTCAGTGGTGTACGGTACAAACCCA
SEQ ID No. 49 pUC19 plasmid sequence

GAGATACCTACAGCGTGAGCTATGAGAAAGCGCCACGCTTCCCGAAGGGAGAAAGGCGGACAGGTATCC
GGTAAGCGGCAGGGTCGGAACAGGAGAGCGCACGAGGGAGCTTCCAGGGGGAAACGCCTGGTATCTTTA
TAGTCCTGTCGGGTTTCGCCACCTCTGACTTGAGCGTCGATTTTTGTGATGCTCGTCAGGGGGGCGGAGCC
TATGGAAAAACGCCAGCAACGCGGCCTTTTTACGGTTCCTGGCCTTTTGCTGGCCTTTTGCTCACATGTTCTT
TCCTGCGTTATCCCCTGATTCTGTGGATAACCGTATTACCGCCTTTGAGTGAGCTGATACCGCTCGCCGCAG
CCGAACGACCGAGCGCAGCGAGTCAGTGAGCGAGGAAGCGGAAGAGCGCCCAATACGCAAACCGCCTCT
CCCCGCGCGTTGGCCGATTCATTAATGCAGCTGGCACGACAGGTTTCCCGACTGGAAAGCGGGCAGTGAG
CGCAACGCAATTAATGTGAGTTAGCTCACTCATTAGGCACCCCAGGCTTTACACTTTATGCTTCCGGCTCGT
ATGTTGTGTGGAATTGTGAGCGGATAACAATTTCACACAGGAAACAGCTATGACCATGATTACGCCAAGCT
TGCATGCCTGCAGGTCGACTCTAGAGGATCCCCGGGTACCGAGCTCGAATTCACTGGCCGTCGTTTTACAA
CGTCGTGACTGGGAAAACCCTGGCGTTACCCAACTTAATCGCCTTGCAGCACATCCCCCTTTCGCCAGCTGG
CGTAATAGCGAAGAGGCCCGCACCGATCGCCCTTCCCAACAGTTGCGCAGCCTGAATGGCGAATGGCGCC
TGATGCGGTATTTTCTCCTTACGCATCTGTGCGGTATTTCACACCGCATATGGTGCACTCTCAGTACAATCTG
CTCTGATGCCGCATAGTTAAGCCAGCCCCGACACCCGCCAACACCCGCTGACGCGCCCTGACGGGCTTGTC
TGCTCCCGGCATCCGCTTACAGACAAGCTGTGACCGTCTCCGGGAGCTGCATGTGTCAGAGGTTTTCACCG
TCATCACCGAAACGCGCGAGACGAAAGGGCCTCGTGATACGCCTATTTTTATAGGTTAATGTCATGATAAT
AATGGTTTCTTAGACGTCAGGTGGCACTTTTCGGGGAAATGTGCGCGGAACCCCTATTTGTTTATTTTTCTA
AATACATTCAAATATGTATCCGCTCATGAGACAATAACCCTGATAAATGCTTCAATAATATTGAAAAAGGAA
GAGTATGAGTATTCAACATTTCCGTGTCGCCCTTATTCCCTTTTTTGCGGCATTTTGCCTTCCTGTTTTTGCTC
ACCCAGAAACGCTGGTGAAAGTAAAAGATGCTGAAGATCAGTTGGGTGCACGAGTGGGTTACATCGAACT
GGATCTCAACAGCGGTAAGATCCTTGAGAGTTTTCGCCCCGAAGAACGTTTTCCAATGATGAGCACTTTTA
AAGTTCTGCTATGTGGCGCGGTATTATCCCGTATTGACGCCGGGCAAGAGCAACTCGGTCGCCGCATACAC
TATTCTCAGAATGACTTGGTTGAGTACTCACCAGTCACAGAAAAGCATCTTACGGATGGCATGACAGTAAG
AGAATTATGCAGTGCTGCCATAACCATGAGTGATAACACTGCGGCCAACTTACTTCTGACAACGATCGGAG
GACCGAAGGAGCTAACCGCTTTTTTGCACAACATGGGGGATCATGTAACTCGCCTTGATCGTTGGGAACCG
GAGCTGAATGAAGCCATACCAAACGACGAGCGTGACACCACGATGCCTGTAGCAATGGCAACAACGTTGC

GCAAACTATTAACTGGCGAACTACTTACTCTAGCTTCCCGGCAACAATTAATAGACTGGATGGAGGCGGAT
AAAGTTGCAGGACCACTTCTGCGCTCGGCCCTTCCGGCTGGCTGGTTTATTGCTGATAAATCTGGAGCCGG
TGAGCGTGGGTCTCGCGGTATCATTGCAGCACTGGGGCCAGATGGTAAGCCCTCCCGTATCGTAGTTATCT
ACACGACGGGGAGTCAGGCAACTATGGATGAACGAAATAGACAGATCGCTGAGATAGGTGCCTCACTGAT
TAAGCATTGGTAACTGTCAGACCAAGTTTACTCATATATACTTTAGATTGATTTAAAACTTCATTTTTAATTT
AAAAGGATCTAGGTGAAGATCCTTTTTGATAATCTCATGACCAAAATCCCTTAACGTGAGTTTTCGTTCCAC
TGAGCGTCAGACCCCGTAGAAAAGATCAAAGGATCTTCTTGAGATCCTTTTTTTCTGCGCGTAATCTGCTGC
TTGCAAACAAAAAAACCACCGCTACCAGCGGTGGTTTGTTTGCCGGATCAAGAGCTACCAACTCTTTTTCCG
AAGGTAACTGGCTTCAGCAGAGCGCAGATACCAAATACTGTTCTTCTAGTGTAGCCGTAGTTAGGCCACCA
CTTCAAGAACTCTGTAGCACCGCCTACATACCTCGCTCTGCTAATCCTGTTACCAGTGGCTGCTGCCAGTGG
CGATAAGTCGTGTCTTACCGGGTTGGACTCAAGACGATAGTTACCGGATAAGGCGCAGCGGTCGGGCTGA
ACGGGGGGTTCGTGCACACAGCCCAGCTTGGAGCGAACGACCTACACCGAACT

**Table 4 Sequence Descriptions**

| SEQ ID No. | Sequence Description |
|---|---|
| SEQ ID No. 1 | Amino acid sequence of integrase Cyin14 |
| SEQ ID No. 2 | Amino acid sequence of integrase Cyin10 |
| SEQ ID No. 3 | Amino acid sequence of integrase Cyin11 |
| SEQ ID No. 4 | Amino acid sequence of integrase Cyin12 |
| SEQ ID No. 5 | Amino acid sequence of integrase Cyin13 |
| SEQ ID No. 6 | Amino acid sequence of integrase Cyin15 |
| SEQ ID No. 7 | Amino acid sequence of integrase Cyin16 |
| SEQ ID No. 8 | Donor plasmid sequence without integration sequence (EGFP donor plasmid) |
| SEQ ID No. 9 | Backbone vector sequence used for integrase expression plasmids |
| SEQ ID No. 10 | Nucleic acid sequence encoding Cyin14 |
| SEQ ID No. 11 | Nucleic acid sequence encoding Cyin10 |
| SEQ ID No. 12 | Nucleic acid sequence encoding Cyin11 |
| SEQ ID No. 13 | Nucleic acid sequence encoding Cyin12 |
| SEQ ID No. 14 | Nucleic acid sequence encoding Cyin13 |
| SEQ ID No. 15 | Nucleic acid sequence encoding Cyin15 |
| SEQ ID No. 16 | Nucleic acid sequence encoding Cyin16 |
| SEQ ID No. 17 | Integration sequence 10-P2 on Cyin10 donor plasmid |
| SEQ ID No. 18 | Integration sequence 10-B2 on Cyin10 donor plasmid |
| SEQ ID No. 19 | Integration sequence 11-P1 on Cyin11 donor plasmid |
| SEQ ID No. 20 | Integration sequence 11-B1 on Cyin11 donor plasmid |
| SEQ ID No. 21 | Integration sequence 11-P2 on Cyin11 donor plasmid |
| SEQ ID No. 22 | Integration sequence 11-B2 on Cyin11 donor plasmid |
| SEQ ID No. 23 | Integration sequence 12-P1 on Cyin12 donor plasmid |
| SEQ ID No. 24 | Integration sequence 12-B1 on Cyin12 donor plasmid |
| SEQ ID No. 25 | Integration sequence 12-P2 on Cyin12 donor plasmid |
| SEQ ID No. 26 | Integration sequence 12-B2 on Cyin12 donor plasmid |
| SEQ ID No. 27 | Integration sequence 13-P1 on Cyin13 donor plasmid |
| SEQ ID No. 28 | Integration sequence 13-B1 on Cyin13 donor plasmid |
| SEQ ID No. 29 | Integration sequence 13-P2 on Cyin13 donor plasmid |
| SEQ ID No. 30 | Integration sequence 13-B2 on Cyin13 donor plasmid |
| SEQ ID No. 31 | Integration sequence 14-P1 on Cyin14 donor plasmid |
| SEQ ID No. 32 | Integration sequence 14-B1 on Cyin14 donor plasmid |
| SEQ ID No. 33 | Integration sequence 14-P2 on Cyin14 donor plasmid |
| SEQ ID No. 34 | Integration sequence 14-B2 on Cyin14 donor plasmid |
| SEQ ID No. 35 | Integration sequence 15-P1 on Cyin15 donor plasmid |
| SEQ ID No. 36 | Integration sequence 15-B1 on Cyin15 donor plasmid |
| SEQ ID No. 37 | Integration sequence 15-P2 on Cyin15 donor plasmid |
| SEQ ID No. 38 | Integration sequence 15-B2 on Cyin15 donor plasmid |
| SEQ ID No. 39 | Integration sequence 16-P1 on Cyin16 donor plasmid |
| SEQ ID No. 40 | Integration sequence 16-B1 on Cyin16 donor plasmid |
| SEQ ID No. 41 | Integration sequence 16-P2 on Cyin16 donor plasmid |
| SEQ ID No. 42 | Integration sequence 16-B2 on Cyin16 donor plasmid |
| SEQ ID No. 43 | Amino acid sequence of phiC31 |
| SEQ ID No. 44 | Amino acid sequence of Bxb1 |
| SEQ ID No. 45 | Nucleotide sequence of phiC31 |
| SEQ ID No. 46 | Nucleotide sequence of Bxb1 |
| SEQ ID No. 47 | Integration sequence attB of integrase phiC31 |
| SEQ ID No. 48 | Integration sequence attP of integrase Bxb1 |
| SEQ ID No. 49 | Sequence of control plasmid pUC19 |

All documents mentioned in the disclosure are incorporated by reference in this application, just as each individual document is incorporated by reference separately. Furthermore, it should be understood that after reading the above teachings of the present disclosure, those skilled in the art can make various changes or modifications to the disclosure, and these equivalent forms also fall within the scope defined by the appended claims of this application.

## Claims

1. An integrase selected from the group consisting of:
(a) an integrase represented by any one of SEQ ID NO: 1-7;
(b) a polypeptide with an amino acid sequence having at least 80%, at least 85%, at least 90%, at least 91%, at least 92%, at least 93%, at least 94%, at least 95%, at least 96%, at least 97%, at least 98%, or at least 99% sequence identity to the amino acid sequence represented by any one of SEQ ID NO: 1-7, and substantially retaining the biological function of any one of SEQ ID NO: 1-7; and
(c) a polypeptide derived from (a), which is formed by substitution, deletion, or addition of one or more amino acid residues in the amino acid sequence represented by any one of SEQ ID NO: 1-7, and has an integrase function.

2. A polynucleotide encoding the integrase according to claim 1.

3. A vector comprising the polynucleotide according to claim 2.

4. The vector according to claim 3, wherein a regulatory sequence includes a promoter sequence.

5. A genetically engineered host cell, containing the vector according to claim 3, or having the polynucleotide according to claim 2 integrated in its genome.

6. A method for preparing the integrase according to claim 1, comprising:
(a) culturing the host cell according to claim 5 under conditions suitable for expression; and
(b) isolating the integrase from the culture.

7. A recombination method, comprising:
(a) providing a target nucleic acid to be integrated, and a host genome into which the target nucleic acid is to be integrated;
(b) in the presence of the integrase according to claim 1, allowing the target nucleic acid to be integrated into the genome.

8. A reaction system for gene integration, comprising:
(a) a target nucleic acid to be integrated;
(b) a host genome, into which the target nucleic acid is to be integrated; and
(c) the integrase according to claim 1.

9. A kit for detecting a target nucleic acid in a sample, comprising:
(a) the integrase according to claim 1;
(b) the polynucleotide according to claim 2; or
(c) the vector according to claim 3.

10. Use of the integrase according to claim 1, the polynucleotide according to claim 2, the vector according to claim 3, or the host cell according to claim 5 in the preparation of a reagent or kit, wherein the reagent or kit is for use in:
(i) gene or genome editing;
(ii) editing a target sequence in a target locus to modify an organism or a non-human organism.

11. A fusion protein, comprising a DNA-binding nuclease and the integrase according to claim 1 or an active fragment thereof.

12. A method for site-specific integration of an exogenous nucleic acid into the genome or an intracellular target nucleic acid of a cell, comprising:
(a) introducing into a cell (i) a first construct, (ii) a gRNA, (iii) an exogenous nucleic acid to be integrated, and (iv) a second construct;
wherein
(i) the first construct is an expressible polynucleotide construct encoding an editing polypeptide, wherein the editing polypeptide comprises a DNA-binding nuclease domain linked to a reverse transcriptase domain via a linker, and the DNA-binding nuclease domain has nickase activity;
(ii) the gRNA is a guide RNA comprising a targeting sequence, a primer binding sequence, and a complementary sequence to an integration sequence;
wherein the gRNA interacts with the expressed editing polypeptide, thereby targeting the editing polypeptide to a specific target site of the genome or the intracellular target nucleic acid of the cell, and the DNA-binding nuclease domain of the editing polypeptide cleaves one strand of the genome or the intracellular target nucleic acid of the cell, and the reverse transcriptase domain incorporates the integration sequence into the cleaved site, thereby incorporating at least one integration sequence into a specific target site of the genome or target nucleic acid of the cell;
(iii) the exogenous nucleic acid to be integrated is linked to a recognition sequence homologous to the integration sequence, and in the presence of both the recognition sequence and the integration sequence, the integrase mediates the site-specific integration;
and
(iv) the second construct is an expressible polynucleotide construct encoding the integrase according to claim 1;
wherein the integrase integrates the exogenous nucleic acid into the specific target site of the genome of the cell;
(c) in the presence of the editing polypeptide, the gRNA, the integrase, and the exogenous nucleic acid to be integrated, the integrase integrates the exogenous nucleic acid into the genome or the intracellular target nucleic acid of the cell.

13. A sequence integration system, comprising:
(i) a first construct, which is an expressible polynucleotide construct encoding an editing polypeptide, wherein the editing polypeptide comprises a DNA-binding nuclease domain linked to a reverse transcriptase domain via a linker, and the DNA-binding nuclease domain has nickase activity;
(ii) a gRNA, which is a guide RNA comprising a targeting sequence, a primer binding sequence, and a complementary sequence to an integration sequence;
wherein the gRNA interacts with the expressed editing polypeptide, thereby targeting the editing polypeptide to a specific target site of the genome or the intracellular target nucleic acid of a cell, and the DNA-binding nuclease domain of the editing polypeptide cleaves one strand of the genome or the intracellular target nucleic acid of the cell, and the reverse transcriptase domain incorporates the integration sequence into the cleaved site, thereby incorporating at least one integration sequence into a specific target site of the genome or target nucleic acid of the cell;
(iii) an exogenous nucleic acid to be integrated, which is linked to a recognition sequence homologous to the integration sequence, and the recognition sequence and the integration sequence can mediate the site-specific integration; and
(iv) a second construct encoding the integrase according to claim 1;
wherein the integrase integrates the exogenous nucleic acid into the specific target site of the genome of the cell.

14. A therapeutic method, comprising integrating an exogenous nucleic acid to be integrated into the genome of a subject in need by using the method according to claim 12.

15. The method according to claim 14, wherein the subject includes a human or a non-human mammal.
